# EUROPÄISCHE PATENTSCHRIFT

(11) **EP 3 684 450 B1**
(45) Veröffentlichungstag und Bekanntmachung des Hinweises auf die Patenterteilung: **02.11.2022**
(21) Anmeldenummer: 18773119.5
(22) Anmeldetag: 13.09.2018
(51) Int. Cl.: G06F 3/0362, G06F 3/04847, A61M 16/00, A61M 16/04, G06F 3/01

(54) **BEATMUNGSVORRICHTUNG MIT BEDIENVORRICHTUNG MIT HAPTISCHER RÜCKKOPPLUNG**
VENTILATOR COMPRISING OPERATING DEVICE HAVING HAPTIC FEEDBACK
DISPOSITIF DE RESPIRATION ARTIFICIELLE MUNI D'UN DISPOSITIF DE COMMANDE À RÉTROACTION HAPTIQUE

(30) Priorität: 22.09.2017 DE 102017122046
(43) Veröffentlichungstag der Anmeldung: 29.07.2020
(73) Patentinhaber: Hamilton Medical AG, 7402 Bonaduz (CH)
(72) Erfinder: HIMMELSTOSS, Matthias, 7000 Chur (CH); LENDENMANN, Urs, 7000 Chur (CH)
(74) Vertreter: Schmitt-Nilson Schraud Waibel Wohlfrom Patentanwälte Partnerschaft mbB
(86) Internationale Anmeldenummer: PCT/EP2018/074791
(87) Internationale Veröffentlichungsnummer: WO 2019/057606

(56) Entgegenhaltungen:
- US-A1- 2010 095 961
- US-A1- 2012 268 285
- US-A1- 2017 209 664

## Beschreibung

Die vorliegende Erfindung betrifft eine Beatmungsvorrichtung zur maschinellen Beatmung von Patienten, umfassend eine Bedienvorrichtung zum Einstellen wenigstens eines Beatmungsparameters mit einem Manipulationsglied, welches zur manuellen Einstellung des wenigstens einen Beatmungsparameters durch eine Bedienperson manuell betätigbar ist. Die vorliegende Erfindung betrifft darüber hinaus ein Verfahren zum Bereitstellen einer unterstützenden Interaktion beim Einstellen wenigstens eines Parameters einer Beatmungsvorrichtung sowie ein Computerprogrammprodukt, das Programmanweisungen enthält, bei deren Ausführung auf der Steuereinheit der erfindungsgemäßen Beatmungsvorrichtung das erfindungsgemäße Verfahren zum Bereitstellen einer unterstützenden Interaktion beim Einstellen wenigstens eines Parameters einer Beatmungsvorrichtung durchgeführt wird.

Die Steuereinheit der Beatmungsvorrichtung ist erfindungsgemäß dazu ausgebildet, auf Grundlage von erfassten Werten von Kenngrößen einen Sollwert für den wenigstens einen Beatmungsparameter automatisiert zu bestimmen.

Es wird vorgeschlagen, dass der Bedienvorrichtung eine erste Haptik-Rückkopplungseinheit zugeordnet ist, die dazu ausgebildet ist, das Manipulationsglied im Sinne einer ersten haptischen Rückkopplung derart anzusteuern, dass eine dem von der Steuereinheit bestimmten Sollwert des wenigstens einen Beatmungsparameters entsprechende Stellung des Manipulationsglieds für die Bedienperson wahrnehmbar hervorgehoben wird.

Erfindungsgemäß ist der Bedienvorrichtung eine erste Haptik-Rückkopplungseinheit zugeordnet, die dazu ausgebildet ist, eine auf Grundlage einer Abweichung zwischen dem durch manuelle Betätigung des Manipulationsglieds aktuell eingestellten Wert des Beatmungsparameters und dem von der Steuereinheit bestimmten Sollwert des Beatmungsparameters bestimmte erste haptische Rückkopplung zu erzeugen. Unter dem Begriff Abweichung soll allgemein eine wie auch immer geartete Abweichung des aktuell eingestellten Werts des Beatmungsparameters von dem Sollwert verstanden werden. Eine solche Abweichung kann beispielsweise als eine Differenz, ein Verhältnis oder eine andere Beziehung zwischen dem aktuell eingestellten Wert und dem Sollwert erfasst werden.

Bislang wird bei Beatmungsvorrichtungen in Regel die Einstellung von Beatmungsparametern so vorgenommen, dass ein oder mehrere Beatmungsparameter durch Manipulation eines Manipulationsglieds, beispielsweise eines Drehknopfs, gemäß einer bestimmten Behandlungssituation, in der sich ein Patient befindet, manuell eingestellt werden oder gemäß einer Veränderungen der Patientensituation nachgestellt werden. Diese Prozedur erfordert häufig mehrere Anpassungsschritte, weil in den meisten Fällen eine Veränderung eines der Beatmungsparameter Einfluss auf andere Beatmungsparameter nimmt und damit eine weitere Anpassung solcher anderer Beatmungsparameter erforderlich wird. Die Anpassung dieser anderen Beatmungsparameter hat dann in der Regel wieder Auswirkung auf den zuerst veränderten Beatmungsparameter, so dass dieser wieder nachgestellt werden muss, usw. Diese Prozedur ist mühsam und erfordert viel Erfahrung. Selbst mit viel Erfahrung ist die korrekte Neueinstellung aller Beatmungsparameter gemäß eines vorliegenden Patientenzustands bzw. bei Veränderung des Patientenzustands zeitaufwändig. Versuche, die bisherigen manuellen Einstellprozeduren zu automatisieren, sind weitgehend erfolglos geblieben, insbesondere weil automatische Prozeduren der Einstellung bzw. Nachführung von Beatmungsparametern durch die Beatmungsvorrichtung häufig vom Bedienungspersonal in Intensivstationen und Krankenhäusern nur wenig akzeptiert werden. Behandelnde Ärzte bzw. Intensivpflegekräfte wollen in aller Regel die Kontrolle über den Beatmungszustand eines Patienten behalten und sind deswegen sehr zurückhaltend gegenüber einer Einführung von automatisierten Prozeduren bei Beatmungsvorrichtungen.

Beispielsweise zeigt die WO 2008/113410 A1 ein Manipulationsglied für manuelles Einstellen eines Beatmungsparameters, insbesondere des Öffnungsdrucks eines Druckbegrenzungsventils in einer Beatmungsvorrichtung. Das Manipulationsglied wird beim Verstellen haptisch beaufschlagt, um das Erreichen eines Grenzwertes für den Beatmungsparameter wahrnehmbar zu machen. Der Grenzwert für den Beatmungsparameter ist fest vorgegeben und die haptische Rückkopplung für das Manipulationsglied wird anhand des Grads einer Verstellung des Manipulationsglieds aus einer Anfangsposition erzeugt.

US 6 834 647 B2 zeigt demgegenüber eine manuell bedienbare Fernsteuerung für eine Beatmungsvorrichtung, durch welche eine manuelle Beatmung durch einen Arzt erfolgen kann, die die Beatmung mittels eines Ambu-Beutels simuliert. Insbesondere soll in der Fernbedienung eine haptische Rückkopplung auf Grundlage eines gemessenen Beatmungsparameters, insbesondere des Atemwegsdrucks, erzeugt werden, die dem Arzt eine fühlbare Rückkopplung über den gerade herrschenden Wert des Beatmungsparameters gibt, entsprechend der Rückkopplung bei manueller Beatmung mittels Ambu-Beutel.

Die US 2010/0095961 A1 beschreibt eine manuelle Eingabe-Ausgabe-Vorrichtung in einem Atemgerät, das eine manuelle Eingabe zum Einstellen mindestens eines patientenkritischen Betriebsparameters des Atemgeräts hat. Die manuelle Eingabe-Ausgabe-Vorrichtung ist für eine haptische Rückmeldung programmierbar und verfügt über ein Bedienelement für die manuelle Eingabe und eine manuelle Ausgabe. Die manuelle Eingabe-Ausgabe-Vorrichtung kann zum Einstellen eines Öffnungsdruckniveaus eines einstellbaren Druckbegrenzungsventils verwendet werden.

Die US 2017/0209664 A1 beschreibt eine Atemunterstützungseinheit zum Bereitstellen von unter Druck stehender, befeuchteter Luft für einen Benutzer, welche ausgebildet ist, die Benutzerverträglichkeit zu erhöhen. Die Atemunterstützungseinheit umfasst Mittel zum Erzeugen positiver emotionaler und kognitiver Zustände eines Benutzers über die Therapie.

Die US 2012/0268285 A1 beschreibt ein System und Verfahren zum Bereitstellen von haptischem Feedback in einem medizinischen Monitor. Das haptische Feedback kann konfigurierbar sein. Beispielsweise kann haptisches Feedback nur einem definierten Satz von Ereignissen zugeordnet sein oder je nach dem Ereignis, das das Auftreten des haptischen Feedbacks auslöst, unterschiedliche Eigenschaften haben.

Die Erfindung löst die Aufgabe, die manuelle Einstellung eines Beatmungsparameters durch eine Bedienperson zu vereinfachen.

Zur Lösung dieser Aufgabe wird eine Beatmungsvorrichtung zur maschinellen Beatmung von Patienten gemäß des Anspruchs 1 vorgeschlagen

Insbesondere kann die Steuereinheit derart ausgebildet sein, dass sie, in der Regel auf Grundlage eines bestimmten Beatmungsmodells, wenigstens eine automatisch arbeitende Steuer/Regelprozedur zur vollautomatischen Beatmung von Patienten abarbeitet. Beispiele solcher teilweise oder weitgehend vollständig automatisch arbeitender Beatmungsprozeduren sind beispielsweise unter den Namen "ASV" und "INTELLIVENT-ASV" bei Beatmungsvorrichtungen der Anmelderin bekannt. Die automatische Beatmungsprozedur arbeitet dabei aber gewissermaßen unsichtbar oder "im Hintergrund". Damit soll zum Ausdruck gebracht werden, dass einer oder mehrere der von der Beatmungsprozedur festgelegten Beatmungsparameter nicht unmittelbar durch die Beatmungsvorrichtung zur Beatmung angewendet werden. Vielmehr sollen diese Beatmungsparameter weiterhin nur manuell einstellbar sein durch manuelle Betätigung des Manipulationsglieds seitens einer Bedienperson. Dennoch sollen die automatisch berechneten Beatmungsparameter, auch wenn sie nicht unmittelbar zur automatischen Beatmung angewendet werden sollen, bei der Beatmung Berücksichtigung finden, und zwar in Sinne einer haptischen Rückkopplung, die auf das Manipulationsglied im Zuge der manuellen Einstellung zurückwirkt.

Die hier vorgeschlagene Lösung erlaubt es, bei manueller Manipulation des Manipulationsglieds eine intuitive Unterstützung durch die Beatmungsvorrichtung bereitzustellen, ohne dadurch die Kontrollmöglichkeiten für die Bedienperson zu beeinträchtigen. Die hier vorgeschlagene Lösung entwickelt den Stand der Technik hinsichtlich intuitiver Bedienbarkeit einer Beatmungseinrichtung weiter, lässt allerdings weiterhin für eine Bedienperson alle möglicherweise gewünschten manuellen Kontrollmöglichkeiten zu. Insbesondere impliziert die hier vorgeschlagene Lösung eine haptische Rückmeldung auf Grundlage eines von der Beatmungsvorrichtung vorgegebenen Beatmungsparameters, oder auf Grundlage eines durch die Beatmungsvorrichtung angewendeten Modells des Patientenzustands und/oder der jeweils einzusetzenden Beatmungsstrategie bzw. des jeweils zu wählenden Beatmungsmodus, aus dem die jeweils optimalen Beatmungsparameter bei dem jeweiligen Patientenzustand ableitbar sind. Das unterstützt das Bedienpersonal darin, teilweise sehr komplexe Zusammenhänge zwischen einzelnen Beatmungsparametern bei manueller Einstellung oder Veränderung eines Beatmungsparameters zu berücksichtigen. Hierfür ist von Bedeutung, dass die hier vorgeschlagene Lösung die Wahrnehmbarkeit solcher Zusammenhänge verbessert, und zwar auf eine sehr intuitive Weise. Insbesondere erlaubt es die hier vorgeschlagene Lösung, solche Zusammenhänge zu erfühlen, insbesondere unmittelbar in Reaktion auf eine manuelle Manipulation des Manipulationsglieds zum Zwecke der Neueinstellung oder Nachjustierung eines der Beatmungsparameter. Solche komplexen Zusammenhänge können beispielsweise Druck-Volumen-Zusammenhänge sein, wie sie bei jeweiligen Einstellungen von Basisgrößen, die den Zustand des Patienten reflektieren sollen, empfohlen sind. Diese Hilfe bei der Einstellung von Beatmungsparametern erhöht letztendlich die Sicherheit bei der künstlichen Beatmung, weil das Bedienpersonal davor gewarnt wird, ungewöhnliche oder jedenfalls nicht gängigen Beatmungsmodellen entsprechende Einstellungen von Beatmungsparametern vorzunehmen.

Unter dem Begriff Sollwert soll sowohl ein einzelner Wert verstanden werden, als auch ein bestimmter Wertebereich oder eine Wertegrenze, etwa eine Obergrenze oder eine Untergrenze, verstanden werden. Mit dem Begriff "wahrnehmbar hervorgehoben" soll zum Ausdruck gebracht werden, dass die Bedienperson im Zuge eines Manipulationsvorgangs des Manipulationsglieds eine von der Beatmungsvorrichtung erzeugte Rückkopplung erfährt, die mittels einer beliebigen Sinneswahrnehmung erkennbar ist. Insbesondere kann die Rückkopplung fühlbar sein, also bei Kontakt mit der Beatmungseinrichtung, insbesondere bei Kontakt mit dem Manipulationsglied, eine spürbare Sinneswahrnehmung auslösen. Man spricht bei Rückkopplungen dieser Art allgemein von haptischer Rückkopplung. Insbesondere soll die haptische Rückkopplung mit demselben Organ erfassbar sein, dass zur Einstellung des Bedienparameters auf das Manipulationsglied einwirkt. Beispielsweise kann im Falle einer manuellen Einstellung des Beatmungsparameters mittels einer Hand des Benutzers eine haptische Rückkopplung auf die Hand, oder jedenfalls ein der Hand zugeordnetes Organ, insbesondere auf einen oder mehrere Finger oder den Handteller, wirken. Die haptische Rückkopplung kann beispielsweise im Sinne eines Widerstandes wirken, den das Manipulationsglied einer weiteren Verstellung in der vom Bediener ausgeübten Richtung entgegensetzt. Der Widerstand, den das Manipulationsglied einer Manipulation des Manipulationsglieds entgegensetzt, kann umso kleiner werden je stärker eine Manipulation des Manipulationsglieds zu einem von der Steuereinheit der Beatmungsvorrichtung bevorzugten Wert des jeweils einzustellenden Beatmungsparameters hin gerichtet ist. Das kleiner Werden kann dabei auch durchaus plötzlich oder abrupt erfolgen, um der Bedienperson das Gefühl eines "Einrastens" zu geben, wenn im Zuge eines Manipulationsvorgangs eine Stellung erreicht wird, die einem von der Steuereinheit der Beatmungsvorrichtung vorgeschlagenen Wert des Beatmungsparameters entspricht. Umgekehrt kann der Widerstand, den das Manipulationsglied einer Manipulation des Manipulationsglieds entgegensetzt, umso größer werden, je stärker eine Manipulation des Manipulationsglieds von einem von der Steuereinheit der Beatmungsvorrichtung bevorzugten Werts des jeweils einzustellenden Beatmungsparameters weg gerichtet ist. Auch hier kann nach Überschreiten eines von der Steuereinheit der Beatmungsvorrichtung vorgeschlagenen Wert des Beatmungsparameters bei weiterer manueller Betätigung des Manipulationsglied eine schlagartige oder abrupte Erhöhung des Widerstands erfolgen.

Die Haptik-Rückkopplungseinheit kann insbesondere einen Antrieb zum Erzeugen einer haptischen Rückkopplung aufweisen, sowie eine Sensorik zur Erfassung eines oder mehrerer Parameter, auf dessen/deren Grundlage die Haptik-Rückkopplung eingestellt wird. Die Haptik-Rückkopplungseinheit kann hierzu eine mit geeigneten Regelalgorithmen ausgebildete Steuer/Regeleinheit aufweisen. Der Antrieb kann zur Erzeugung einer geeigneten haptischen Rückkopplung, beispielsweise in Form einer Rückkopplungskraft, eines Rückkopplungsmoments, und/oder einer fühlbaren Rückkopplungsbewegung, ausgebildet sein. Die genannten Effekte können einzeln ausgeprägt sein, in vielen Fällen wird es aber günstig sein, mindestens zwei dieser Effekte zu überlagern. Eine haptische Rückkopplungskraft oder ein haptisches Rückkopplungsmoment kann beispielsweise eine Änderung eines einer Verstellung des Manipulationsglieds entgegen wirkenden Widerstands bei zunehmender oder abnehmender Verstellung des Manipulationsglieds bewirken. Eine haptische Rückkopplungsbewegung kann in Form einer verstellungsabhängigen fühlbaren Bewegung des Manipulationsglieds erfolgen, insbesondere in Form einer Vibration bei Erreichen oder in der Nähe einer Sollstellung des Manipulationsglieds. Beispielsweise eignet sich als Antrieb für die Haptik-Rückkopplungseinheit ein Servoantrieb mit geeigneter Regelung von Lage, Drehzahl und/oder Drehmoment auf Grundlage von vorbestimmten Eingangsparametern. Beispielsweise erfüllen elektronisch geregelte Servoantriebe hohe bis sehr hohen dynamische Anforderungen und sind in der Lage, eine auf das Manipulationsglied einwirkende geeignete haptische Rückkopplung unmittelbar in Reaktion auf Erfassen eines oder mehrerer Beatmungsparameter und/oder auf Erfassen einer Veränderung der Stellung des Manipulationsglieds zu erzeugen. Geeignete Sensorik zur Erfassung des einen oder der mehreren Beatmungsparameter und/oder zur Erfassung der Stellung bzw. einer Verstellung des Manipulationsglieds aus einer Anfangsstellung wird in vielen Fällen ohnehin vorhanden sein und kann ohne Weiteres zur Erzeugung der haptischen Rückkopplung herangezogen werden. Lediglich beispielhaft sei verwiesen auf Sensorik zur Erfassung von Druck und/oder Gasfluss in Atemwegsleitungen von der Beatmungsvorrichtung zum Patienten und/oder weg vom Patienten, oder Sensorik zur Erfassung von Sauerstoffsättigung im arteriellen Blut mittels Pulsoxymetrie oder anderen Methoden. Sensorik zur Erfassung einer aktuellen Stellung des Manipulationsglieds bzw. zur Erfassung einer Verstellung der Manipulationsglieds aus einer Anfangsstellung kann beispielsweise auf Grundlage von elektrischen und/oder magnetischen Feldern, die bei Betätigung des Manipulationsglieds entstehen oder sich verändern, realisiert werden. Auch solche Sensorik zur Erfassung einer Stellung oder Verstellung des Manipulationsglieds wird in aller Regel ohnehin vorhanden sein.

In diesem Zusammenhang ist darauf hinzuweisen, dass eine mit Betätigung des Manipulationsglieds einhergehende Verstellung des Manipulationsglieds aus einer Anfangsstellung nicht unbedingt sofort als geänderter Wert des jeweiligen Beatmungsparameters in der Steuerung/Regelung der von der Beatmungsvorrichtung durchgeführten Beatmung des Patienten Anwendung finden muss. In vielen Fällen ist es sogar wünschenswert, dass eine mit Verstellung des Manipulationsglieds einhergehende Änderung des jeweiligen Beatmungsparameters sich solange noch nicht in der Beatmung auswirkt, bis der jeweils neu eingestellte Parameterwert bestätigt worden ist. Diese Bestätigung kann seitens der Beatmungsvorrichtung geschehen, beispielsweise nachdem die Beatmungsvorrichtung erfasst hat, dass das Manipulationsglied nach Verstellung über eine vorbestimmte Zeitdauer hinweg ohne weitere Verstellung in einer neuen Stellung verblieben ist und die Beatmungsvorrichtung so konfiguriert ist, dass sie diese neue Stellung dann als gewünschte Endstellung ansieht. Dann ist eine entsprechende Rückmeldung an den Anwender, dass der neu eingestellte Parameterwert von der Beatmungsvorrichtung übernommen worden ist, sinnvoll, etwa akustisch, visuell oder auch haptisch. Oft wird man aber zur Übernahme eines mittels Verstellen des Manipulationsglieds neu eingestellten Parameterwerts eine Bestätigung durch den Anwender selbst verlangen, etwa indem der Anwender das Manipulationsglied entsprechend betätigt oder ein weiteres Manipulationsglied betätigt. Beispielsweise kann im Falle eines als Dreh-/Drücksteller ausgebildeten Manipulationsglieds ein Verstellen eines Beatmungsparameters durch Verdrehen des Manipulationsglieds aus einer Anfangsstellung bis zu einer dem neuen Wert entsprechenden Stellung erfolgen, während eine Bestätigung eines neu eingestellten Werts ein nachfolgendes, zusätzliches Niederdrücken des Manipulationsglieds verlangt. Erst nach dem Bestätigen wird der neu eingestellte Wert für den jeweiligen Beatmungsparameter von der Beatmungsvorrichtung in der Beatmung des Patienten übernommen und fortan angewendet. Bei solcher Konfiguration ist es sinnvoll, die hier angesprochene Haptik-Rückkopplung auf Grundlage einer Verstellung des Manipulationsglieds aus einer Anfangsstellung zu erzeugen (und nicht auf Grundlage eines tatsächlich bei der Beatmung angewendeten Parameterwerts), weil der verstellte Parameterwert noch nicht von der Beatmungsvorrichtung übernommen worden ist und sich die mit der Verstellung des Manipulationsglieds einhergehende Veränderung des jeweiligen Beatmungsparameters ja noch nicht auf die Beatmung auswirkt. Wenn also im Rahmen der vorliegenden Offenbarung von einem "aktuell eingestellten Wert" eines Beatmungsparameters die Rede ist, so ist damit gemeint ein einer Verstellung des Manipulationsglieds entsprechender Wert des jeweiligen Beatmungsparameters, wie er sich einstellen würde, wenn der gerade eingestellte Wert bestätigt und von der Beatmungsvorrichtung übernommen werden würde. Die Haptik-Rückkopplung beruht also auf einer Simulation der Beatmung nach Maßgabe der aktuellen Verstellung des Manipulationsglieds und gibt ggf. sogar eine solche simulierte Beatmungssituation wieder. Dies gilt gleichermaßen für die oben bereits angesprochene erste Haptik-Rückkopplung also auch für die nachfolgend noch zu erläuternde zweite Haptik-Rückkopplung.

Insbesondere kann die Steuereinheit dazu ausgebildet sein, den Sollwert für den wenigstens einen Beatmungsparameter fortlaufend, insbesondere synchronisiert mit jeweiligen Atemzyklen, automatisiert zu bestimmen. Damit stellt die Steuereinrichtung eine im Hintergrund ablaufende teilweise oder vollständig, jedenfalls weitgehend vollständig, automatisch arbeitende Beatmungsprozedur bereit, die fortlaufend, insbesondere mit den Atemzyklen synchronisiert, alle relevanten Beatmungsparameter dem gerade herrschenden Patientenzustand angepasst einstellt bzw. nachstellt. Die Steuereinheit wäre im Prinzip in der Lage, alle relevanten Beatmungsparameter fortlaufend anzupassen, so dass manuelle Einstellungen bzw. Nachstellungen dieser Beatmungsparameter an sich überflüssig wären.

Wie bereits angesprochen, ist es günstig, wenn die erste Haptik-Rückkopplungseinheit dazu ausgebildet ist, das Manipulationsglied derart anzusteuern, dass die dem von der Steuereinheit bestimmten Sollwert des wenigstens einen Beatmungsparameters entsprechende Stellung des Manipulationsglieds für die Bedienperson fühlbar hervorgehoben wird. Dies kann beispielsweise durch eine Rückmeldung geschehen, die der Bedienperson ein "Klick"-Gefühl oder "Einrast"-Gefühl vermittelt, wenn ein von der Steuereinheit bestimmter Sollwert erreicht oder überstrichen wird. Denkbar wäre etwa eine abrupte Veränderung eines Widerstands, den das Manipulationsglied einer Manipulationsbewegung entgegensetzt kurz vor Erreichen des Sollwerts, insbesondere eine abrupte Verringerung dieses Widerstands. Dementsprechend würde sich der Widerstand dann kurz nach Überstreichen des Sollwerts wieder abrupt in umgekehrter Richtung verändern, also insbesondere wieder abrupt ansteigen. Andere haptische Rückmeldungen sind ebenfalls denkbar, wie unten näher ausgeführt.

Erfindungsgemäß ist die erste Haptik-Rückkopplungseinheit dazu ausgebildet, eine auf Grundlage einer Abweichung zwischen dem durch manuelle Betätigung des Manipulationsglieds aktuell eingestellten Wert des Beatmungsparameters und dem von der Steuereinheit bestimmten Sollwert des Beatmungsparameters bestimmte erste haptische Rückkopplung zu erzeugen.

Erfindungsgemäß wird eine Beatmungsvorrichtung zur maschinellen Beatmung von Patienten vorgeschlagen, die Folgendes umfasst: eine Bedienvorrichtung zum Einstellen wenigstens eines Beatmungsparameters mit einem Manipulationsglied, welches zur manuellen Einstellung des wenigstens einen Beatmungsparameters durch eine Bedienperson manuell betätigbar ist, und eine Steuereinheit, welche dazu ausgebildet ist, auf Grundlage von erfassten Werten von Kenngrößen einen Sollwert für den wenigstens einen Beatmungsparameter automatisiert zu bestimmen, wobei der Bedienvorrichtung eine erste Haptik-Rücckopplungseinheit zugeordnet ist, die dazu ausgebildet ist, eine auf Grundlage einer Abweichung zwischen dem durch manuelle Betätigung des Manipulationsglieds aktuell eingestellten Wert des Beatmungsparameters und dem von der Steuereinheit bestimmten Sollwert des Beatmungsparameters bestimmte erste haptische Rückkopplung zu erzeugen.

Wie bereits eingangs angesprochen, soll unter dem Begriff Abweichung allgemein eine wie auch immer geartete Abweichung des aktuell eingestellten Werts des Beatmungsparameters von dem Sollwert verstanden werden. Eine solche Abweichung kann beispielsweise als eine Differenz, ein Verhältnis oder eine andere Beziehung zwischen dem aktuell eingestellten Wert und dem Sollwert erfasst werden. Zur Umsetzung dieser Ausgestaltung kann als Abweichung eine Funktion gebildet werden, die eine die Abweichung ausdrückende Beziehung herstellt zwischen dem im Zuge eines Manipulationsvorgangs am Manipulationsglied aktuell eingestellten Wert des Beatmungsparameters und dem jeweils aktuell von der Steuereinheit berechneten oder abgeleiteten Sollwert für diesen Beatmungsparameter. Anhand dieser Funktion kann eine haptische Rückkopplung erzeugt werden. Diese haptische Rückkopplung kann die ihr zu Grunde liegende Funktion direkt wiedergeben. Alternativ ist auch denkbar, dass dieser Funktion noch eine weitere Größe überlagert wird, beispielsweise ein abrupte Änderung der haptischen Rückkopplung bei Erreichen des Sollwerts (in der Regel wenn die Funktion einen Extremwert, insbesondere einen Minimalwert, erreicht), um ein Klick-Gefühl zu erzeugen, wie oben bereits ausführlich dargelegt.

Es sei nochmals darauf hingewiesen, dass mit dem Begriff "aktuell eingestellter Wert" eines Beatmungsparameters ein einer gerade vorgenommenen Verstellung des Manipulationsglieds entsprechender Wert des jeweiligen Beatmungsparameters gemeint ist, wie er sich einstellen würde, wenn der gerade eingestellte Wert bestätigt und von der Beatmungsvorrichtung übernommen werden würde.

Weitere optionale Ausgestaltungen, die für alle Aspekte der vorliegenden Erfindung gelten, sollen im Folgenden näher dargelegt werden. Es versteht sich, dass jede dieser optionalen Ausgestaltungen für sich alleine zu den vorangehend ausgeführten Ausgestaltungen hinzutreten kann, dass die angesprochenen optionalen Ausgestaltungen jedoch auch in beliebiger Kombination miteinander mit den oben diskutierten Ausgestaltungen kombiniert werden können.

In manchen Fällen kann es günstig sein, wenn das Manipulationsglied eine Tendenz hat, von selbst eine Stellung einzunehmen, die dem durch die Steuereinheit bestimmten Sollwert des Beatmungsparameters entspricht. Dann kann man vorsehen, dass die erste Haptik-Rückkopplungseinheit das Manipulationsglied in einer solchen Weise beaufschlagt, dass das Manipulationsglied ohne manuelle Betätigung eine Stellung einnimmt - oder jedenfalls einzunehmen sucht -, die dem durch die Steuereinheit bestimmten Sollwert des Beatmungsparameters entspricht. Ohne Betätigung durch eine Bedienperson, oder wenn eine solche Betätigung nicht zu Ende geführt wird, stellt das Manipulationsglied dann autonom den von der Steuereinheit vorgegeben Wert für den Beatmungsparameter ein. Selbstverständlich kann einer solchen haptischen Rückkopplung noch eine weitere gewünschte haptische Rückkopplung - etwa das oben diskutierte Klick-Gefühl - überlagert werden. Letztendlich könnte man bei einer solchen Ausgestaltung sogar eine vollständig autonome Einstellung des Beatmungsparameters durch die Steuereinheit realisieren. Wichtig ist aber, dass die Bedienperson jederzeit die Möglichkeit hat, durch manuelle Betätigung einen anderen Wert für den Beatmungsparameter einzustellen als den von der Steuereinheit vorgegebenen.

Hierbei kann unterstützend, aber auch unabhängig von den vorangehend besprochenen Varianten, auch vorgesehen sein, dass die erste Haptik-Rückkopplungseinheit das Manipulationsglied in einer solchen Weise beaufschlagt, dass das Manipulationsglied in eine Stellung zurückkehrt - oder jedenfalls in eine solche Stellung zurückzukehren sucht -, die dem durch die Steuereinheit bestimmten Sollwert des Beatmungsparameters entspricht, wenn nach erfolgter manueller Betätigung das Manipulationsglied wieder freigegeben wird. Ein solches Verhalten des Manipulationsglieds braucht nicht notwendigerweise über den gesamten Verstellbereich des Manipulationsglieds hinweg vorgesehen zu sein. Es genügt vielmehr, wenn das Manipulationsglied sich jedenfalls in einer Umgebung um den Sollwert herum so verhält. Dann führt nämlich die die erste haptische Rückkopplung die Bedienperson zum von der Steuereinheit vorgegebenen Sollwert hin, sobald sich das Manipulationsglied im Zuge eines Manipulationsvorgangs einen vorgegebenen Bereich um den Sollwert herum erreicht. Sobald sich das Manipulationsglied in diesem Bereich befindet, zentriert sich das Manipulationsglied infolge der ersten haptischen Rückkopplung beim durch die Steuereinheit vorgegebenen Sollwert, zumindest solange keine manuelle Betätigung erfolgt.

Beispielsweise kann die erste haptische Rückkopplung das Manipulationsglied mit einer Rückstellkraft oder einem Rückstellmoment mit der Charakteristik einer um eine Ruhestellung ausgelenkten Feder, insbesondere einer um einen Ruhepunkt ausgelenkten harmonischen Feder, beaufschlagen. Diese Charakteristik kann jedenfalls in dem zuvor erwähnten Bereich um den Sollwert herum vorgesehen sein. Sie kann beispielsweise mit einer weiteren haptischen Rückkopplung kombiniert sein. Beispielsweise kann vorgesehen sein, dass die erste haptische Rückkopplung mit einer zweiten haptischen Rückkopplung kombiniert ist, die einen der gerade gewählten Stellung des Manipulationsglieds entsprechenden Wert des Beatmungsparameters anzeigt.

Wie bereits angesprochen kann die erste Haptik-Rückkopplungseinheit dazu ausgebildet sein, im Zuge eines manuellen Manipulationsvorgangs des Manipulationsglieds bei Überfahren einer Stellung des Manipulationsglieds, die dem von der Steuereinheit bestimmten Sollwert des Beatmungsparameters entspricht, eine abrupte Änderung der ersten haptischen Rückkopplung zu erzeugen. Damit soll gemeint sein, dass eine abrupte Änderung eines Rückkopplungsparameters erfolgt, der die erste haptische Rückkopplung charakterisiert. Dies kann insbesondere eine Rückkopplungskraft oder ein Rückkopplungsmoment sein. Unter dem Begriff abrupt soll eine starke, insbesondere eine sprunghafte oder unstetige Veränderung des Werts für den Rückkopplungsparameter verstanden werden, beispielsweise eine sprunghafte oder unstetige Veränderung, oder jedenfalls eine zumindest näherungsweise sprunghafte oder unstetige Veränderung, einer Rückkopplungskraft oder eines Rückkopplungsmoments, die/das zur Weiterführung der manuellen Betätigung des Manipulationsglieds überwunden werden muss. Der Rückkopplungsparameter soll insbesondere bei oder kurz vor Erreichen der dem Sollwert entsprechenden Stellung des Manipulationsglieds abrupt kleiner werden und nach Überschreiten der dem Sollwert entsprechenden Stellung abrupt größer werden, so dass ein Einrastgefühl oder Klick-Gefühl bei Überstreichen des der dem Sollwert entsprechenden Stellung wahrnehmbar (insbesondere erfühlbar) ist.

In manchen Fällen kann es - je nach dem gewählten einzustellenden Beatmungsparameter - nützlich sein, wenn die erste Haptik-Rückkopplung davon abhängig ist, in welchem von mehreren Wertebereichen für den Beatmungsparameter sich die Abweichung des durch manuelle Betätigung des Manipulationsglieds aktuell eingestellten Wertes des Beatmungsparameters von dem von der Steuereinheit bestimmten Sollwert des Beatmungsparameters befindet. Man kann auf diese Weise ohne Weiteres mehrere von der Steuereinheit bevorzugte Sollwerte für den Beatmungsparameter haben, wobei die erste haptische Rückkopplung sich jeweils auf den Sollwert für den Beatmungsparameter bezieht, der einem jeweiligen Bereich zugeordnet ist, in dem sich das Manipulationsglied gerade befindet. Wechselt dieser Bereich im Zuge eines Manipulationsvorgangs, so wechselt dann auch der jeweils aktuell gültige Sollwert.

Dabei können die mehreren Wertebereiche für den Beatmungsparameter durch jeweilige Wertegrenzen voneinander getrennt sein und die Steuereinheit dazu ausgebildet sein, die Wertegrenzen zwischen den jeweiligen Wertebereichen für den Beatmungsparameter fortlaufend, insbesondere synchronisiert mit jeweiligen Atemzyklen, zu bestimmen. Auf diese Weise kann die jeweilige intuitive Unterstützung einer Bedienperson bei Einstellung eines oder mehrerer Beatmungsparameter automatisch an eine Veränderung des Patientenzustands angepasst werden.

Zur Erzeugung der ersten haptischen Rückkopplung, insbesondere zur Bestimmung der Abweichung zwischen dem durch manuelle Betätigung des Manipulationsglieds aktuell eingestellten Wert des Beatmungsparameters und dem von der Steuereinheit bestimmten Sollwert des Beatmungsparameters, kann der ersten Haptik-Rückkopplungseinheit wenigstens ein Sensor zugeordnet sein, der zur Erfassung der Stellung des Manipulationsglieds, insbesondere in Bezug auf eine Referenzstellung, ausgebildet ist. Ein solcher Sensor zur Erfassung der aktuellen Stellung oder Position des Manipulationsglieds wird häufig ohnehin vorgesehen sein. Dann kann die Steuereinheit anhand einer vorgegebenen Zuordnung einer aktuellen Stellung des Manipulationsglieds zu einem jeweils dieser Stellung entsprechenden Wert des Beatmungsparameters die Abweichung zwischen dem durch manuelle Betätigung des Manipulationsglieds aktuell eingestellten Wert des Beatmungsparameters und dem von der Steuereinheit bestimmten Sollwert des Beatmungsparameters bestimmen und die haptische Rückkopplung entsprechend einstellen.

Um der Bedienperson ein noch besseres Gefühl hinsichtlich des gerade eingestellten, oder hinsichtlich des gerade einzustellenden, Werts für den Beatmungsparameters zu vermitteln, kann man die vorangehend besprochene erste Haptik-Rücckopplung noch mit einer zweiten Haptik-Rückkopplung kombinieren, die den aktuellen Ist-Zustand des Beatmungsparameters anzeigt, oder jedenfalls den aktuellen Ist-Zustand des Beatmungsparameters bei Aktivierung des aktuell durch Manipulation des Manipulationsglieds eingestellten Werts. Hierzu kann dem Manipulationsglied eine zweite Haptik-Rückkopplungseinheit zugeordnet sein, welche das Manipulationsglied abhängig von einem Ist-Zustand des wenigstens einen Beatmungsparameters im Sinne einer zweiten haptischen Rückkopplung beaufschlagt. Die erste und die zweite haptische Rückkopplung können dabei einander überlagert sein. Die erste und die zweite haptische Rückkopplung können insbesondere einen gemeinsamen Widerstand gegenüber einer (ggf. weiteren) Bewegung des Manipulationsglieds bilden. Dabei müssen die erste und die zweite Haptik-Rückopplungseinheit nicht unbedingt vollständig voneinander unabhängige Bauteile sein. Vielmehr können die erste und die zweite Haptik-Rückkopplungseinheit gemeinsame Bauteile aufweisen oder durch dieselben Bauteile gebildet sein. Insbesondere kann ein ein gemeinsamer Antrieb, der auf das Manipulationsglied wirkt, zur Erzeugung der ersten und zweiten haptischen Rückkopplung dienen.

Insbesondere kann die zweite Haptik-Rückkopplungseinheit das Manipulationsglied mit einer den Ist-Zustand des wenigstens einen Beatmungsparameters anzeigenden zweiten haptischen Rückkopplung beaufschlagen. Dann ergibt sich insgesamt eine für die Bedienperson fühlbare Haptik-Rückkopplung, die einer Überlagerung aus erster Haptik-Rückkopplung und zweiter Haptik-Rückkopplung entspricht. Die Bedienperson kann somit zwei Informationen aus der Haptik-Rücckopplung entnehmen: Zum Einen, wie sich der jeweils einzustellende Beatmungsparameter mit weiterer Verstellung des Manipulationsglied verändern würde und zum Anderen auch, welcher Wert des Beatmungsparameters der in der aktuellen Situation von der Steuereinheit ausgewählte Wert wäre.

Auch der zweiten Haptik-Rückkopplungseinheit kann wenigstens ein Sensor zugeordnet sein, der zur Erfassung des wenigstens einen Beatmungsparameters ausgebildet ist. Der der zweiten Haptik-Rückkopplungseinheit zugeordnete Sensor kann auch gleichzeitig ein der ersten Haptik-Rückkopplungseinheit zugeordnetes Signal liefern, aus dem die Stellung des Manipulationsglieds in Bezug auf eine vorbestimmte Referenzstellung, insbesondere in Bezug auf die dem von der Steuereinheit bestimmten Sollwert des Beatmungsparameters entsprechende Stellung des Manipulationsglieds, erfassbar ist.

Der Sensor kann insbesondere zur Erfassung wenigstens einer der folgenden Kenngrößen vorgesehen sein: aktueller Atemwegsdruck am Atemwegseingang, aktueller Gasfluss am Atemwegseingang, kumulierter Gasfluss im Atemweg während der Inspirationsphase eines Atemzyklus, kumulierter Gasfluss im Atemweg während der Expirationsphase eines Atemzyklus, Kohlendioxidkonzentration im vom Patienten am Ende eines Atemzyklus ausgeatmeten Atemgas, arterielle Sauerstoffsättigung im Blutkreislauf des Patienten. Der Sensor kann insbesondere so ausgebildet sein, dass er die jeweilige Kenngröße kontinuierlich, oder quasi kontinuierlich erfasst, so dass sich dann aus den vom Sensor gelieferten Daten der aktuell eingestellte Wert des jeweils gewünschten Beatmungsparameters (beispielsweise des maximalen endexpiratorischen Drucks PEEP) ermitteln lässt. Es ist aber auch denkbar, den Sensor so auszubilden, dass die vom Sensor gelieferten Daten auf den jeweils gewünschten Beatmungsparameter abgestimmt sind (beispielsweise der Sensor den PEEP unmittelbar am Ende eines Atemzyklus bestimmt). Selbstverständlich können zur Erfassung mehrerer Kenngrößen mehrere Sensoren vorgesehen sein, die jeweils zur Erfassung einer oder mehrerer der Kenngrößen vorgesehen sind.

Die Steuereinheit kann insbesondere dazu ausgebildet sein, auf Grundlage der erfassten Werte von Kenngrößen einen Beatmungsmodus aus einer Mehrzahl von vorgegebenen Beatmungsmodi auszuwählen und auf Grundlage des ausgewählten Beatmungsmodus den Sollwert für den wenigstens einen Beatmungsparameter automatisiert zu bestimmen. Beispielsweise kann aus dem kumulierter Gasfluss im Atemweg während der Expirationsphase eines Atemzyklus eine Beatmungs-Zeitkonstante RC abgeleitet werden, und anhand dieser Beatmungs-Zeitkonstante RC der Beatmungsmodus festgelegt werden. Es sind eine Vielzahl aus von Beatmungsstrategien oder Beatmungsmodi bekannt, die je nach Patientenzustand angewendet werden. Es sind auch Beatmungssysteme bekannt, die automatisch, d.h. im Wesentlichen ohne manuelle Eingriffe, je nach erfasste Kenndaten, die den Zustand eines Patienten charakterisieren, zwischen verschiedenen Beatmungsmodi umschalten können. Als ein Beispiel sei auf das von der Anmelderin vertriebene Beatmungssystem "ASV" (Adapative Support Ventilation) oder "INTELLIVENT-ASV" verwiesen.

Der wenigstens eine einzustellende Beatmungsparameter kann beispielsweise wenigstens einen der folgenden Parameter umfassen: Beatmungsfrequenz, Tidalvolumen, Minutenvolumen, Inspirationszeit, positiver endexpiratorischer Druck (PEEP), maximaler Atemwegsdruck, Sauerstoffkonzentration im dem Patienten zugeführten Atemgas. Es ist auch denkbar, mehrere dieser Parameter zu kombinieren, wobei man dann in der Regel die mehren einzustellenden Parameter nacheinander mit Hilfe des hier vorgeschlagenen Systems einstellen wird.

Das Manipulationsglied kann als Einstellvorrichtung beliebiger Art ausgestaltet sein, beispielsweise als beweglicher Griff, Drehhebel, Kipphebel, Wippe oder dergleichen. Auch Eingabevorrichtungen in Form einer Tastatur, einer Maus, eines Trackballs, eines Touchpad, eines Tablets oder dergleichen sind denkbar. Häufig wird das Manipulationsglied als Drehglied ausgebildet sein oder ein als Drehglied ausgebildetes Einstellelement umfassen. Ein solches Drehglied kann insbesondere als Drehknopf ausgebildet sein, der gegen die erste und ggf. zweite Haptik-Rückkopplung manuell drehbar ist. Die erste und ggf. zweite Haptik-Rückkopplung stellt dann ein der jeweils gewünschten Rückkopplung entsprechendes Gegen-Drehmoment bereit, das zur (weiteren) Betätigung des Drehglieds überwunden werden muss. Solche Drehknöpfe sind bei vielen Beatmungsvorrichtungen zur manuellen Einstellung von Beatmungsparametern vorgesehen. Man kann in solchen Fällen im Prinzip die vorhandene Hardware des Manipulationsglieds weiter nutzen und muss nur dafür sorgen, dass das Manipulationsglied entsprechend mit der ersten Haptik-Rückkopplung und ggf. mit der zweiten Haptik-Rückkopplung beauf schlagt wird.

In bestimmten Ausführungsformen kann das Manipulationsglied als Dreh-/Drück-Steller ausgebildet sein, so dass durch unterschiedliche Art der Betätigung unterschiedliche Beatmungsparameter eingestellt werden können. Generell kann das Manipulationsglied zur Einstellung von mehreren Beatmungsparametern ausgebildet sein. Dies kann beispielsweise realisiert werden durch Vorauswahl des jeweils gewünschten Beatmungsparameters über ein weiteres Manipulationsglied, z.B. einen Touchscreen, eine Tastatur, oder weiteres Dreh- oder Drückelement, wobei dann für den jeweils gewählten Beatmungsparameter der gewünschte Wert mit Hilfe des eigentlichen Manipulationsglieds eingestellt wird. Im Falle von Dreh-/Drück-Stellern kann die Vorauswahl eines oder mehrerer Beatmungsparameter durch entsprechende Einstellung des Manipulationsglieds selbst erfolgen, z.B. durch Einstellen des Manipulationsglieds in eine vorbestimmte Kippstellung oder Translationsstellung zur Vorauswahl des Beatmungsparameters und Drehbewegung des Manipulationsglieds zur Einstellung des gewünschten Parameterwerts.

Gemäß einem weiteren Aspekt betrifft die vorliegende Erfindung auch ein Verfahren zum Bereitstellen einer unterstützenden Interaktion beim Einstellen wenigstens eines Parameters einer Beatmungsvorrichtung, bei welchem ein Manipulationsglied einer Bedienvorrichtung zum Einstellen des wenigstens einen Parameters durch eine Bedienperson manuell betätigt wird, und auf Grundlage von erfassten Werten von Kenngrößen ein Sollwert für den wenigstens einen Parameter durch eine Steuereinheit automatisiert bestimmt wird, wobei das Manipulationsglied während der manuellen Betätigung im Sinne einer ersten haptischen Rückkopplung derart angesteuert wird, dass eine dem von der Steuereinheit bestimmten Sollwert des wenigstens einen Parameters entsprechende Stellung des Manipulationsglieds für die Bedienperson wahrnehmbar, insbesondere fühlbar, hervorgehoben wird.

Alternativ betrifft die vorliegende Erfindung auch ein Verfahren zum Bereitstellen einer unterstützenden Interaktion beim Einstellen wenigstens eines Parameters bei einer Beatmungsvorrichtung, bei welchem ein Manipulationsglied einer Bedienvorrichtung zum Einstellen des wenigstens einen Parameters durch eine Bedienperson manuell betätigt wird, und auf Grundlage von erfassten Werten von Kenngrößen ein Sollwert für den wenigstens einen Parameter durch eine Steuereinheit automatisiert bestimmt wird, wobei das Manipulationsglied während der manuellen Betätigung des Manipulationsglieds mit einer auf Grundlage einer Abweichung zwischen dem durch manuelle Betätigung des Manipulationsglieds aktuell eingestellten Wert des Parameters und dem von der Steuereinheit bestimmten Sollwert des Parameters bestimmten ersten haptischen Rückkopplung beaufschlagt wird.

Die hier vorgeschlagenen Verfahren betreffen nicht die Bedienung einer Beatmungsvorrichtung an sich, sondern richten sich vielmehr darauf, dass die Beatmungsvorrichtung Informationen für Bedienpersonen auf intuitiv erfassbare Weise zu Verfügung stellt. Diese Bereitstellung von intuitiv erfassbaren Informationen durch die Beatmungsvorrichtung erfolgt dabei automatisiert und ohne jedes Zutun seitens der Bedienperson. Insbesondere sind keinerlei Eingaben seitens der Bedienperson erforderlich. Vielmehr ist die Beatmungsvorrichtung dazu ausgelegt, derartige Information im Wege einer ersten und ggf. zweiten haptischen Rückkopplung automatisch und ohne weiteres Zutun seitens der Bedienperson zu erzeugen. Dies gilt für alle nachfolgend noch etwas näher erläuterten Verfahrensschritte.

Diese Verfahren können sinngemäß alle Merkmale umfassen, die vorangehend mit Bezug auf eine Beatmungsvorrichtung beschrieben worden sind, so dass auf die entsprechende Beschreibung verwiesen werden kann.

Insbesondere kann bei den hier vorgeschlagenen Verfahren das Manipulationsglied durch die erste und ggf. zweite Haptik-Rückkopplungseinheit in einer solchen Weise beaufschlagt werden, dass das Manipulationsglied ohne manuelle Betätigung eine Stellung einnimmt - oder jedenfalls einzunehmen sucht -, die dem durch die Steuereinheit bestimmten Sollwert des Beatmungsparameters entspricht, oder dass das Manipulationsglied in eine Stellung zurückkehrt - oder jedenfalls in eine solche Stellung zurückzukehren sucht -, die dem durch die Steuereinheit bestimmten Sollwert des Beatmungsparameters entspricht, wenn nach erfolgter manueller Betätigung das Manipulationsglied wieder freigegeben wird. Es erfolgt dann also eine vollautomatische Einstellung des jeweiligen Beatmungsparameters entsprechend dem von der Beatmungsvorrichtung vorgeschlagenen Wert.

Das Manipulationsglied kann durch die erste und ggf. zweite Haptik-Rückkopplungseinheit mit einer Rückstellkraft oder einem Rückstellmoment mit der Charakteristik einer um eine Ruhestellung ausgelenkten Feder, insbesondere einer um einen Ruhepunkt ausgelenkten harmonischen Feder, beaufschlagt werden.

Insbesondere kann bei den beschriebenen Verfahren im Zuge eines manuellen Manipulationsvorgangs des Manipulationsglieds bei Überfahren einer Stellung des Manipulationsglieds, die dem von der Steuereinheit bestimmten Sollwert des Beatmungsparameters entspricht, durch die erste und ggf. zweite Haptik-Rückkopplungseinheit automatisiert eine abrupte Änderung der ersten haptischen Rücckopplung zu erzeugt werden, insbesondere eine solche haptische Rückmeldung,d ein "Einrasten" oder ein "Klick-Gefühl" erzeugt.

Die erste haptische Rückkopplung kann bei dem Verfahren davon abhängig sein, in welchem von mehreren Wertebereichen für den Beatmungsparameter sich die Abweichung des durch manuelle Betätigung des Manipulationsglieds aktuell eingestellten Wertes des Beatmungsparameters von dem von der Steuereinheit bestimmten Sollwert des Beatmungsparameters befindet. Dabei können die mehreren Wertebereiche für den Beatmungsparameter durch jeweilige Wertegrenzen voneinander getrennt sein und die Steuereinheit dazu ausgebildet sein, die Wertegrenzen zwischen den jeweiligen Wertebereichen für den Beatmungsparameter fortlaufend, insbesondere synchronisiert mit jeweiligen Atemzyklen, zu bestimmen.

Wie oben beschrieben kann bei dem Verfahren das Manipulationsglied durch die zweite Haptik-Rückkopplungseinheit zusätzlich zu der durch die erste Haptik-Rückkopplungseinheit erzeugten ersten haptischen Rückkopplung abhängig von einem Ist-Zustand des wenigstens einen Beatmungsparameters im Sinne einer zweiten haptischen Rückkopplung beaufschlagt werden. Dabei kann das Manipulationsglied mit einer den Ist-Zustand des wenigstens einen Beatmungsparameters anzeigenden zweiten haptischen Rückkopplung beaufschlagt werden.

In einen weiteren Aspekt betrifft die vorliegende Erfindung auch ein Computerprogrammprodukt, welches Programmanweisungen enthält, bei deren Ausführung auf einer Datenverarbeitungsanlage ein Verfahren ausgeführt wird, wie es hierin beschrieben ist.

Die Erfindung wird im Folgenden anhand von Ausführungsbeispielen mit Bezug auf die Figuren näher erläutert. Dabei zeigt:
Figur 1: Eine Ansicht einer Beatmungsvorrichtung gemäß einer Ausführungsform.
Figur 2: Eine Ansicht einer Beatmungsvorrichtung gemäß einer weiteren Ausführungsform.
Figur 3: Ein Blockdiagramm, welches wesentliche Funktionseinheiten in einer zur automatisierten Bestimmung von Beatmungsparametern ausgebildeten Steuereinheit einer Beatmungsvorrichtung gemäß Fig. 1 oder 2 darstellt, gemäß einem ersten Beatmungsmodus "ASV".
Figur 4: Ein Blockdiagramm, welches wesentliche Funktionseinheiten in einer zur automatisierten Bestimmung von Beatmungsparametern ausgebildeten Steuereinheit einer Beatmungsvorrichtung gemäß Fig. 1 oder 2 darstellt, gemäß einem weiteren Beatmungsmodus "INTELLIVENT-ASM".
Figur 5: In schematischer Ansicht den Verlauf eines Widerstands gegenüber einer Verstellbewegung eines Manipulationsglieds im Zuge einer manuellen Einstellung eines Beatmungsparameters gemäß einer Ausführungsform.
Figur 6: In schematischer Ansicht den Verlauf eines Widerstands gegenüber einer Verstellbewegung eines Manipulationsglieds im Zuge einer manuellen Einstellung eines Beatmungsparameters gemäß einer weiteren Ausführungsform.

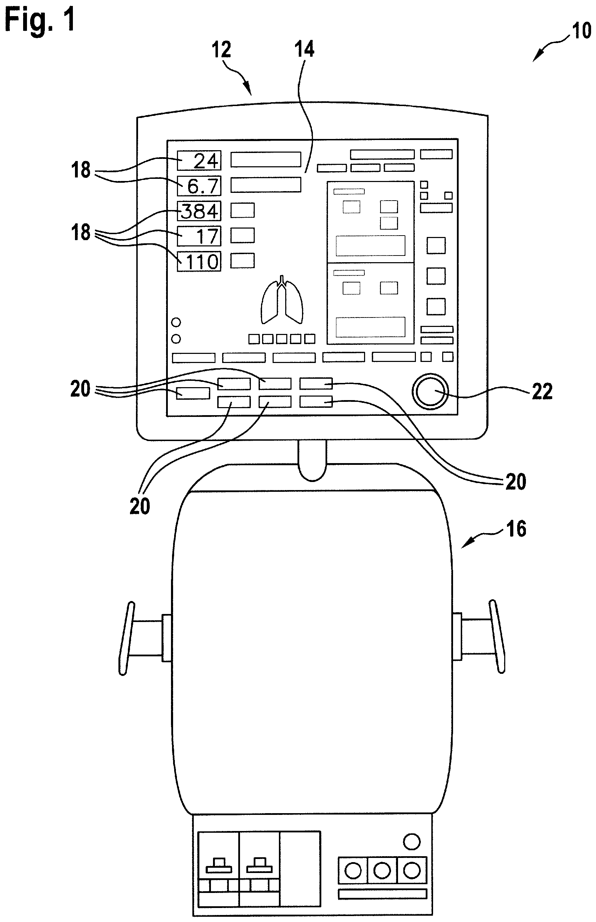

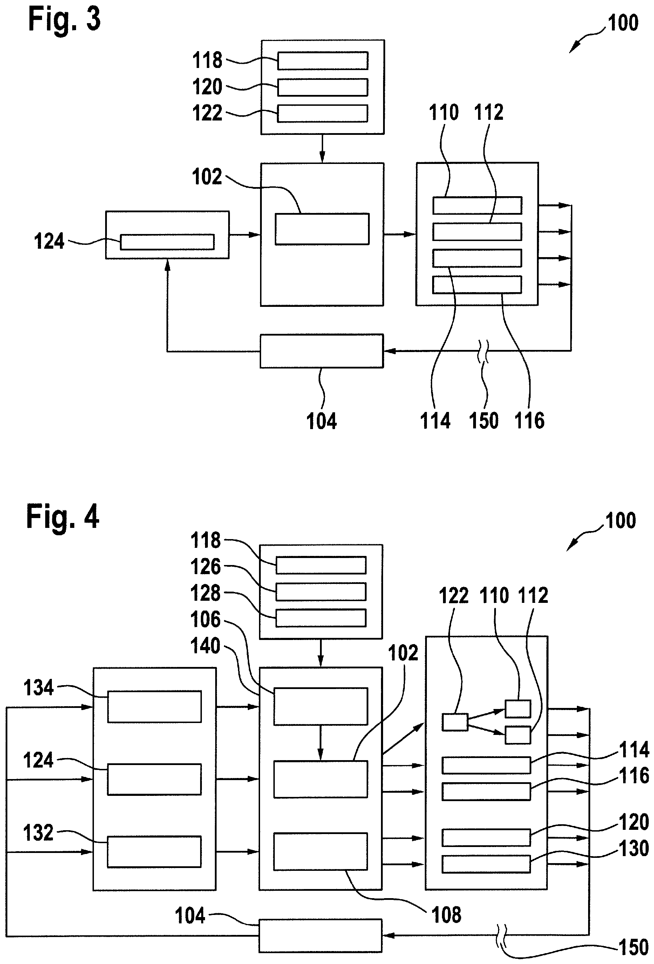

Figur 1 zeigt eine Ansicht einer Beatmungsvorrichtung 10 gemäß einer Ausführungsform. Insbesondere weist die Beatmungsvorrichtung 10 eine Bedieneinheit 12 mit Bildschirm 14 auf, die oberhalb einer Haupteinheit 16 angeordnet ist. Die Haupteinheit 16 umfasst ein Gehäuse, in welchem die erforderlichen Vorrichtungen zur Erzeugung eines Atemgasstroms, insbesondere Pumpen, Ventile, Gasversorgungseinrichtungen und dergleichen, untergebracht sind. Die Haupteinheit 16 enthält zudem eine nicht näher dargestellte Steuereinheit (siehe Figur 3 und 4 für eine schematische Darstellung wesentlicher Funktionsblöcke dieser Steuereinheit), in welcher alle zur Beatmung erforderlichen Steuerprogramme und Routinen gespeichert sind und welche die jeweils zur maschinellen Beatmung erforderlichen Steuerprogramme und Routinen abarbeitet. Der Bildschirm 14 weist eine Mehrzahl von in Figur 1 jeweils mit 18 bezeichnete Anzeigefeldern auf, in denen vorbestimmte Beatmungsparameter dargestellt werden. Zu diesen gehören für einen jeweiligen Beatmungsmodus vorgegebene Beatmungsparameter wie Beatmungsfrequenz, Tidalvolumen, Minutenvolumen, Inspirationszeit, positiver endexpiratorischer Druck (PEEP), maximaler Atemwegsdruck, Sauerstoffkonzentration im dem Patienten zugeführten Atemgas. Auch Werte für bestimmte physiologische Parameter oder den Beatmungszustand kennzeichnende Parameter, die von Sensoren gemessen werden, können dort dargestellt sein wie aktueller Atemwegsdruck am Atemwegseingang, aktueller Gasfluss am Atemwegseingang, kumulierter Gasfluss im Atemweg während der Inspirationsphase eines Atemzyklus, kumulierter Gasfluss im Atemweg während der Expirationsphase eines Atemzyklus, Kohlendioxidkonzentration im vom Patienten am Ende eines Atemzyklus ausgeatmeten Atemgas, arterielle Sauerstoffsättigung im Blutkreislauf des Patienten. Außerdem sind noch graphische Anzeigefelder vorhanden, die in Figur 1 nicht im Einzelnen mit Bezugszeichen versehen sind. In diesen graphischen Anzeigefeldern sind weitere Informationen zur laufenden Beatmungsprozedur dargestellt, etwa eine schematische Darstellung der Lunge, aus der Informationen zu physiologischen Parametern wie Resistance R oder Compliance C wenigstens qualitativ ersichtlich sind, oder graphische Darstellungen des zeitlichen Verlaufs bestimmter sich über einen Atemzyklus hinweg charakteristisch veränderlicher Größen wie Puls, Blutdruck, Atemwegsdruck, Sauerstoffsättigung im But o.ä.

Zudem weist die Bedieneinheit 12 eine Mehrzahl von Eingabefeldern auf, die in Fig. 1 jeweils mit 20 bezeichnet sind. Über diese Eingabefelder 20 können bestimmte Befehle eingegeben werden, beispielsweise Ausdrucke der gerade am Bildschirm 14 angezeigten Funktionen, Versetzen der Beatmungsvorrichtung in Standby-Modus, Bildschirm sperren, Alarmsignal unterdrücken, Beatmung mit reinem Sauerstoff, u.ä. Funktionen. Die Eingabefelder 20 können als mechanische, elektrische oder optische Eingabevorrichtungen ausgebildet sein, wie Tasten, Knöpfe, Schalter und dergleichen. In manchen Ausführungsformen kann der Bildschirm 14 als Touchscreen ausgebildet sein, so dass Eingabe über die Eingabefelder 20 seitens einer Bedienperson durch Berühren des Bildschirms 14 eingegeben werden können. Es kann auch vorgesehen sein, dass bestimmte Eingaben durch Berühren des Bildschirms 14 innerhalb der nicht bezeichneten graphischen Anzeigefelder gemacht werden können.

Neben den genannten Bedien- und Anzeigeelementen 18, 20 weist die Bedieneinheit 12 noch einen Hauptbedienknopf 22 auf. Dieser Hauptbedienknopf 22 dient der manuellen Einstellung von Beatmungsmodi sowie der manuellen Einstellung von Beatmungsparametern durch eine Bedienperson. Der Hauptbedienknopf 22 ist damit ein Beispiel für ein Manipulationsglied, welches zur manuellen Einstellung eines Beatmungsparameters durch eine Bedienperson manuell betätigbar ist. Der Hauptbedienknopf 22 ist in der gezeigten Ausführungsform als Dreh-/Drücksteller ausgebildet. Eine Bedienperson kann nach Auswahl eines bestimmten Beatmungsparameters, z.B. durch Berühren des jeweiligen dem auszuwählenden Beatmungsparameter zugeordneten Bedienfeldes 18, durch Verdrehen des Hauptbedienknopfes 22 einen jeweils eingestellten Wert für den Beatmungsparameter verändern und damit einen neuen gewünschten Wert einstellen. Nach Abschluss des Einstellvorgangs drückt die Bedienperson auf den Hauptbedienknopf (d.h. die Bedienperson führt eine translatorische Betätigung des Hauptbedienknopfs durch), um den neu eingestellten Wert zu bestätigen. Nach erfolgter Bestätigung wird die Beatmung mit dem neu eingestellten Beatmungsparameter, beispielsweise einem höheren oder niedrigeren Wert für den positiven endexpiratorischen Druck PEEP, weitergeführt.

Während des Einstellvorgangs kann die Bedienperson den sich verändernden Wert des neu einzustellenden Beatmungsparameters im jeweiligen Anzeigefeld 18 des Bildschirms 14 erkennen. Die Beatmungsvorrichtung 10 gemäß der hier beschriebenen Art erlaubt aber noch eine weit bequemere und intuitivere Art der Rückmeldung eines einzustellenden Beatmungsparameters, nämlich durch eine haptische Rückkopplung, die auf den Hauptbedienknopf 22 im Zuge einer Manipulationsbewegung zurückwirkt. In dem gezeigten Beispiel mit als Dreh-/Drücksteller ausgebildetem Hauptbedienknopf 22 wirkt die haptische Rückkopplung als ein Widerstand oder ein Gegen-Drehmoment, der durch den Hauptbedienknopf 22 gegenüber einer von einer Bedienperson erzwungenen Verdrehbewegung aus einer Ausgangsstellung A in Richtung zu einer Endstellung E ausgeübt wird.

Es versteht sich, dass eine entsprechende haptische Rückkopplung auch bei einer Umkehrung der Bewegungsrichtung auf den Hauptbedienknopf 22 ausgeübt wird. Kehrt die Bedienperson die Drehrichtung am Hauptbedienknopf 22 im Zuge eines Manipulationsvorgangs um, so kehrt sich auch die Richtung der haptischen Rücckopplung um, d.h. der nunmehr in Rückwärtsrichtung erfolgenden Betätigung des Hauptbedienknopfs (Drehung in umgekehrter Richtung) wird wieder ein entsprechender Widerstand entgegensetzt.

Der Hauptbedienknopf 22 lässt sich also nach einmal erfolgter Auswahl eines Beatmungsparameters nicht mehr frei drehen, sondern nur mit einer gewissen Kraft bzw. einem gewissen Drehmoment, das zur Überwindung der durch die haptische Rückkopplung ausgeübten Kraft bzw. Drehmoments erforderlich ist. Zur Erzeugung der haptischen Rückkopplung weist die Beatmungsvorrichtung 10 eine nicht im Einzelnen in den Figuren gezeigte Haptik-Rückkopplungseinheit auf, die beispielsweise einen auf eine Drehachse des Hauptbedienknopfs 22 wirkenden Servomotor und diesem zugeordnete Sensorik zur Erfassung der aktuellen Drehstellung des Hauptbedienknopfs 22 umfasst. Der Servomotor 22 kann beispielsweise durch die Steuereinheit 100 der Beatmungsvorrichtung 10 angesteuert werden, der normalerweise auch die von der Sensorik der Haptik-Rückkopplungseinheit gelieferten Werte, neben den von weiteren Sensoren der Beatmungsvorrichtung 10 gelieferten Werten, zugeführt werden.

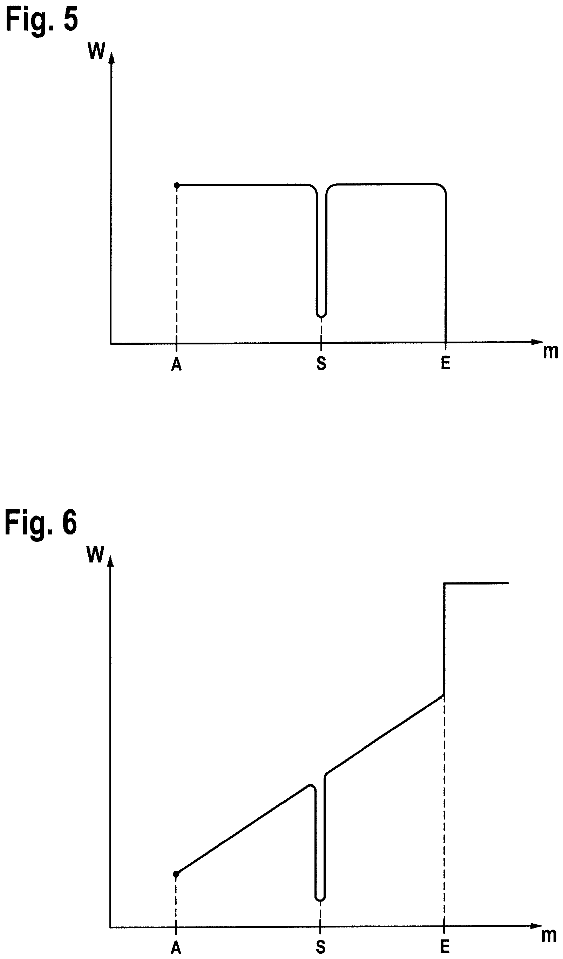

Die von der Haptik-Rückkopplungseinheit gelieferte Haptik-Rückkopplung umfasst mindestens eine erste Haptik-Rückkopplung (siehe Figur 5), die dazu ausgebildet ist, den Hauptbedienknopf 22 im Sinne einer ersten haptischen Rückkopplung derart anzusteuern, dass eine dem von der Steuereinheit bestimmten Sollwert des wenigstens einen Beatmungsparameters entsprechende Stellung des Hauptbedienknopfes 22 für die Bedienperson wahrnehmbar hervorgehoben wird. Alternativ oder zusätzlich kann die erste Haptik-Rückkopplungseinheit dazu ausgebildet sein, den Hauptbedienknopf 22 derart anzusteuern, dass die dem von der Steuereinheit bestimmten Sollwert des wenigstens einen Beatmungsparameters entsprechende Stellung des Hauptbedienknopfes 22 für die Bedienperson fühlbar hervorgehoben wird.

Zusätzlich kann die Haptik-Rückkopplungseinheit noch eine zweite Haptik-Rücckopplungseinheit umfassen, die eine zweite haptische Rückmeldung liefert, die den Hauptbedienknopf 22 abhängig von einem Ist-Zustand des wenigstens einen Beatmungsparameters beaufschlagt. Die zweite haptische Rückkopplung ergibt damit ein fühlbares Signal entsprechend der Verstellung des Hauptbedienknopfs 22 im Zuge einer Manipulation, das die damit einhergehende Veränderung des einzustellenden Beatmungsparameters erfühlbar werden lässt.

Insgesamt ergibt sich dann eine haptische Rückkopplung, die einer Überlagerung der ersten haptischen Rückkopplung mit der zweiten haptischen Rückkopplung entspricht, wie dies in Figur 6 gezeigt ist und mit Bezug zu Figur 6 nachfolgend noch näher erläutert wird.

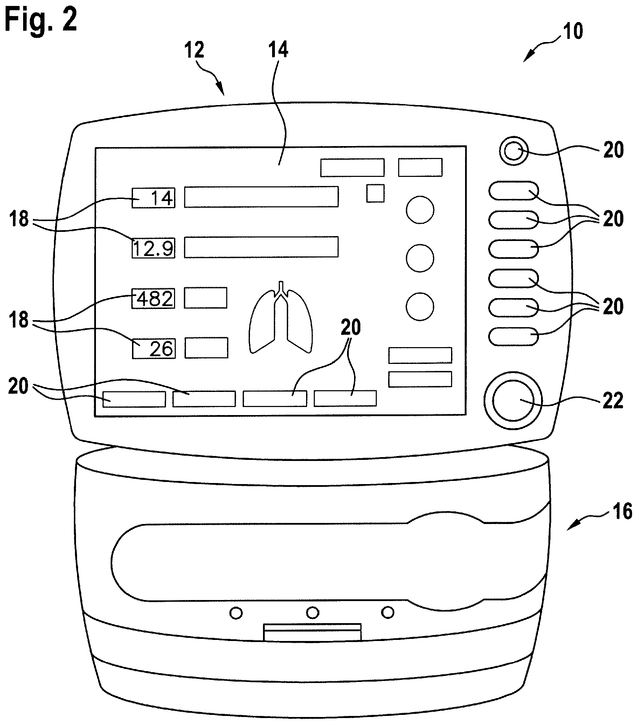

Figur 2 zeigt eine Ansicht einer Beatmungsvorrichtung 10 gemäß einer weiteren Ausführungsform. Die Ansicht gemäß Figur 2 entspricht im Wesentlichen Figur 1. Gleiche Komponenten, oder jedenfalls Komponenten, die dieselbe Funktion haben wie in Figur 1, sind mit denselben Bezugszeichen versehen. Hinsichtlich einer Beschreibung kann auf die Figur 1 verwiesen, die insoweit ohne Weiteres auf in Bezug zu Figur 2 gilt, es sei denn es ist ausdrücklich etwas anderes gesagt.

Fig. 3 zeigt ein Blockdiagramm, welches wesentliche Funktionseinheiten in einer zur automatisierten Bestimmung von Beatmungsparametern ausgebildeten Steuereinheit 100 einer Beatmungsvorrichtung 10 gemäß Fig. 1 oder 2 darstellt, gemäß einem ersten Beatmungsmodus "ASV" 102. Bei diesem Beatmungsmodus stellt die Steuereinheit 100 bestimmte Beatmungsparameter wie beispielsweise die Beatmungsfrequenz 110, das Tidalvolumen 112, die Dauer eines Inspirationsyklus 114 sowie den Beatmungsmodus 116 automatisch ein, während andere Beatmungsparameter wie beispielsweise das Minutenvolumen (hierunter versteht man das Produkt aus Tidalvolumen und Beatmungsfrequenz) 120 oder der positive endexpiratorische Druck PEEP 122 manuell eingestellt bzw. vorgegeben werden. Außerdem werden manuell noch bestimmte physiologische Parameter des zu beatmenden Patienten vorgegeben, beispielsweise dessen Größe 118. Die eigentliche Beatmung 104 wird dabei auf Grundlage der mittels der ASV Prozeduren ermittelten Beatmungsparameter 110 bis 116 sowie einigen der manuell vorgegebenen Parameter 120, 122 durchgeführt, wobei während der Beatmung noch bestimmte physiologische Parameter gemessen werden (in Fig. 2 ist beispielhaft der Atemgasfluss 124 genannt), welche dann ebenfalls als Grundlage für die automatische Festlegung der anderen Beatmungsparameter 110 bis 116 mittels ASV 104 dienen.

Figur 4 zeigt ein weiteres Blockdiagramm, welches wesentliche Funktionseinheiten in einer zur automatisierten Bestimmung von Beatmungsparametern ausgebildeten Steuereinheit 100 einer Beatmungsvorrichtung gemäß Fig. 1 oder 2 darstellt, gemäß einem noch weiter automatisierten Beatmungsmodus "INTELLIVENT-ASM" 140. Der Beatmungsmodus 140 gemäß Figur 4 arbeitet weitgehend automatisiert und umfasst neben bereits mit Bezug zu Figur 3 erläuterten Prozeduren des ASV-Modus 102 noch Prozeduren zur Steuerung von Ventilation 106 und Prozeduren zur Steuerung von Oxygenierung 108. Bei dem Beatmungsmodus 140 stellt die Steuereinheit 100 praktisch alle relevanten Beatmungsparameter weitgehend automatisiert ein, wie beispielsweise das Minutenvolumen 120, das sich wiederum aufteilt in Beatmungsfrequenz 110 und das Tidalvolumen 112, die Dauer eines Inspirationsyklus 114, den Beatmungsmodus 116, sowie mittels der Oxygenierungsprozeduren 106 auch den PEEP 120 und den Sauerstoffgehalt des Beatmungsgases 130. Als Eingangsgröße für die Berechnung dieser Beatmungsparameter dienen im Wesentlichen während der Beatmung 104 erfasste physiologische Parameter wie Atemgasfluss 124, CO2-Gehalt der ausgeatmeten Luft 134 oder Sauerstoffgehalt im arteriellen Blut des Patienten. Diese Parameter werden den Routinen 104, 106, 108 für die automatische Festlegung der anderen Beatmungsparameter 110, 112, 114, bis 116, 120 ,122, 130 als Eingangsgrößen zugeführt. Außerdem werden manuell noch bestimmte physiologische Parameter des zu beatmenden Patienten vorgegeben, beispielsweise dessen Größe 118 oder bestimmte Erkrankungen 126, oder bestimmte Strategien zur Entwöhnung des Patienten von der Beatmung 128. Damit läuft die Beatmung in diesem Beatmungsmodus an sich völlig automatisiert ab und erfordert an sich keine manuellen Eingriffe seitens einer Bedienperson.

Die vorliegende Erfindung gestattet es nun, teilweise oder sogar weitgehend automatisiert ablaufende Beatmungsprozeduren wie ASV 102 oder INTELLIVENT-ASV 140 lediglich als Hilfsmittel zur Unterstützung von Bedienpersonen bei der manuellen Einstellung von Beatmungsparametern zur mechanischen Beatmung einzusetzen. Die Möglichkeit, die Beatmung völlig ohne manuelle Eingriffe durchzuführen, wird also bewusst zurückgestellt und stattdessen wird einer Bedienperson eine möglichst leicht erfassbare und nicht störende Unterstützung bei der korrekten Einstellung von Beatmungsparametern "per Hand" angeboten. Aus diesem Grund werden die jeweils durch die automatischen Beatmungsprozeduren ASV 102 bzw. INTELLIVENT-ASV 140 ermittelten Werte der Beatmungsparameter 110 - 116 bzw. 110, 112, 114, bis 116, 120 ,122, 130 nicht automatisch der eigentlichen Beat mung 104 zugrunde gelegt, sondern es wird zuvor die Möglichkeit angeboten, diese Beatmungsparameter manuell mittels Manipulation des Hauptbedienknopfs 22 einzustellen. Dies ist in den Figuren 3 und 4 dadurch angedeutet, dass die Verbindung zwischen den ermittelten Beatmungsparametern 110 - 116 bzw. 110, 112, 114, bis 116, 120 , 122, 130 und der Beatmung 104 jeweils an der mit 150 bezeichneten Stelle unterbrochen ist. Hier findet also eine Möglichkeit der manuellen Eingabe von Beatmungsparametern statt, bei der die automatisiert ermittelten Werte ggf. auch ganz anders eingestellt werden können. Man kann das so ausdrücken, dass die Beatmungseinrichtung zwar über ganz oder weitgehend vollständig automatisierte Beatmungsmodi verfügt und diese auch bereitstellt, allerdings nicht zur Automatisierung der eigentlich stattfindenden Beatmung, sondern lediglich im Hintergrund als eine Unterstützung für Bedienpersonen bei der manuellen Einstellung dieser Beatmungsparameter. Diese Unterstützung soll durch eine haptische Rücckopplung erfolgen, welche auf ein zur manuellen Einstellung der Beatmungsparameter zu betätigendes Manipulationsglied zurückwirkt.

Weil die im Hintergrund ablaufenden teilweise oder weitgehend automatischen Beatmungsmodi laufend bevorzugte Werte für die jeweils einzustellenden Beatmungsparameter liefern, kann diese haptische Rückkopplung in einer solchen Weise erfolgen, dass die jeweilige Bedienperson eine fühlbare Rückmeldung erhält, wenn sie einen Wert für einen der Beatmungsparameter einstellt, die mit dem von der im Hintergrund ablaufenden Routine eingestellten Wert übereinstimmt.

Die Haptik-Rückkopplung umfasst damit mindestens eine erste Haptik-Rückkopplung (siehe Figur 5), die dazu ausgebildet ist, das den Hauptbedienknopf 22 im Sinne einer ersten haptischen Rückkopplung derart anzusteuern, dass eine dem von der Steuereinheit bestimmten Sollwert des wenigstens einen Beatmungsparameters entsprechende Stellung des Hauptbedienknopfes 22 für die Bedienperson wahrnehmbar hervorgehoben wird. Alternativ oder zusätzlich kann die erste Haptik-Rückkopplungseinheit dazu ausgebildet sein, den Hauptbedienknopf 22 derart anzusteuern, dass die dem von der Steuereinheit bestimmten Sollwert des wenigstens einen Beatmungsparameters entsprechende Stellung des Hauptbedienknopfes 22 für die Bedienperson fühlbar hervorgehoben wird.

Zusätzlich kann die Haptik-Rückkopplungseinheit noch eine zweite Haptik-Rücckopplungseinheit umfassen, die eine zweite haptische Rückmeldung liefert, die den Hauptbedienknopf 22 abhängig von einem Ist-Zustand des wenigstens einen Beatmungsparameters beaufschlagt. Die zweite haptische Rückkopplung ergibt damit ein fühlbares Signal entsprechend der Verstellung des Hauptbedienknopfs 22 im Zuge einer Manipulation, das die damit einhergehende Veränderung des einzustellenden Beatmungsparameters erfühlbar werden lässt.

Insgesamt ergibt sich dann eine haptische Rückkopplung, die einer Überlagerung der ersten haptischen Rückkopplung mit der zweiten haptischen Rückkopplung entspricht, wie dies in Figur 6 gezeigt ist.

Figur 5 zeigt in schematischer Ansicht den Verlauf eines Widerstands W gegenüber einer Verstellbewegung um einen Winkel m des Hauptbedienknopfs 22 im Zuge einer manuellen Einstellung eines der Beatmungsparameter 110 bis 116 bzw. 110, 112, 114, 116, 120, 122, 130 gemäß einer Ausführungsform, bei der nur eine erste haptische Rückmeldung vorhanden ist. Die erste haptische Rückmeldung erzeugt ein fühlbares Klick-Gefühl oder Einrast-Gefühl, wenn die Stellung des Hauptbedienknopfs 22 im Zuge einer Manipulationsbewegung zur Einstellung eines neuen Werts für einen der Beatmungsparameter 110 bis 116 bzw. 110, 112, 114, 116, 120, 122, 130 eine Stellung S erreicht bzw. überschreitet, die dem von der im Hintergrund ablaufenden Routine 102 bzw. 140 automatisch bestimmten Wert entspricht. Wird im Zuge einer Verstellbewegung, beispielsweise ausgehend von einer Anfangsstellung A des Hauptbedienknopfs 22 aus in Richtung zu einer Endstellung E des Hauptbedienknopfs 22 hin, diese dieser Stelle S erreicht, verringert sich nämlich ein einer weiteren Verstellbewegung entgegengesetzter Widerstand W (beispielsweise in Form eines von der Haptik-Rückkopplungseinheit ausgeübten der Verstellbewegung entgegen wirkenden Gegen-Drehmoments) abrupt. Sobald diese Stelle S überschritten ist, erhöht sich der Widerstand wieder genauso abrupt, was dem Bediener das Gefühl eines Einrastens oder Klickens gibt, wenn der Hauptbedienknopf 22 die Stellung S erreicht bzw. überschreitet, die dem von der im Hintergrund ablaufenden Routine 102 bzw. 140 automatisch bestimmten Wert entspricht.

Zusätzlich kann noch vorgesehen sein, dass der Widerstand W dann abrupt auf Null oder nahezu Null zurückgeht, wenn eine Endstellung E erreicht ist, die einem maximal höchstens zulässigen oder einem minimal mindestens erforderlichen Wert des einzustellenden Parameters entspricht. Damit stellt sich beim Bediener ein "Leerlaufgefühl" ein, wenn die Endstellung E erreicht ist, so dass der Bediener ohne Weiteres merkt, dass eine weitere Verdrehung des Hauptbedienknopfs 22 in derselben Richtung keine weitere Veränderung des eingestellten Werts für den Beatmungsparameter mehr bringen wird.

Die Darstellung in Figur 6 entspricht der in Figur 5. Gezeigt ist also der Verlauf des Widerstandes W gegenüber einer Weiterbewegung in derselben Richtung als Funktion einer Verlagerung des Hauptbedienkopfs 22 um einen Winkel m zwischen einer Anfangsstellung A und einer maximal zulässigen Endstellung E. Bei der in Figur 6 gezeigten Ausführungsform ist zusätzlich zu der der mit Bezug zu Figur 5 bereits erläuterten ersten haptischen Rückkopplung bei Erreichen der Sollstellung S zusätzlich noch eine zweite haptische Rückkopplung der ersten haptischen Rückkopplung überlagert. Die zweite haptische Rückkopplung zeigt eine Veränderung des einzustellenden Beatmungsparameters mit weiter zunehmender Verstellung des Hauptbedienknopfs 22 an, beispielsweise einen Anstieg des positiven endexpiratorischen Drucks PEEP mit zunehmender Verstellung m des Hauptbedienknopfs 22. Damit erhält die Bedienperson bereits im Zuge einer Verstellung des Hauptbedienknopfs 22 eine fühlbare Rückmeldung, in welcher Weise sich der gerade ausgewählte Beatmungsparameter verändern wird, wenn die Neueinstellung wirksam geworden ist. Gerade erfahrene Bedienpersonen sind in der Lage, bereits anhand einer solchen fühlbaren Rückmeldung eine gute Abschätzung eines geeigneten Werts für den Parameter vorzunehmen. Ein Vorteil ist, dass die hier vorgeschlagene Vorgehensweise es der Bedienperson ermöglicht, den von ihr erfühlten oder abgeschätzten Wert für den Beatmungsparameter mit dem von der automatisierten Prozedure 102 bzw. 140 vorgeschlagenen Wert zu vergleichen. Außerdem besteht die Möglichkeit, den neuen Wert zunächst nur einzustellen durch Verdrehen des Hauptbedienknopfes 22 bis zur gewünschten Stellung und erst nach erfolgter Einstellung in der Beatmung wirksam werden zu lassen (etwa durch Drücken des Hauptbedienknopfs 22). Man kann also nochmals nachjustieren, wenn beispielsweise einmal eine große Diskrepanz zwischen dem von der Bedienperson erfühlten idealen Wert des Beatmungsparameters und dem von der automatisierten Routine 102 bzw. 140 vorgeschlagenen Wert auftritt.

Bei der Ausführungsform gemäß Figur 6 ist vorgesehen, dass der Widerstand W abrupt auf einen sehr hohen Wert ansteigt, wenn eine Endstellung E erreicht ist, die einem maximal höchstens zulässigen oder einem minimal mindestens erforderlichen Wert des einzustellenden Parameters entspricht. Damit stellt sich beim Bediener das Gefühl eines Anschlags oder einer Endbegrenzung, gegen die der Hauptbedienknopf 22 nicht mehr weiter verdreht werden kann. Auch auf diese Weise lässt sich erreichen, dass die Bedienperson den Hauptbedienknopf 22 nicht über einer Endstellung E hinaus verdrehen kann. Selbstverständlich kann die in Figur 5 gezeigte Variante bei Erreichen der Endstellung E in der Ausführungsform gemäß Figur 6 Anwendung finden, falls gewünscht. Umgekehrt kann selbstverständlich die in Figur 6 gezeigte Variante bei Erreichen der Endstellung E in der Ausführungsform gemäß Figur 5 Anwendung finden, falls gewünscht

## Patentansprüche

1. Beatmungsvorrichtung (10) zur maschinellen Beatmung von Patienten, umfassend
- eine Bedienvorrichtung zum Einstellen wenigstens eines Beatmungsparameters (110, 112, 114, 116; 112, 110, 112, 114, 116, 120, 122, 130) mit einem Manipulationsglied (22), welches zur manuellen Einstellung des wenigstens einen Beatmungsparameters (110, 112, 114, 116; 112, 110, 112, 114, 116, 120, 122, 130) durch eine Bedienperson manuell betätigbar ist, und
- eine Steuereinheit (100), welche dazu ausgebildet ist, auf Grundlage von erfassten Werten von Kenngrößen einen Sollwert für den wenigstens einen Beatmungsparameter (110, 112, 114, 116; 112, 110, 112, 114, 116, 120, 122, 130) automatisiert zu bestimmen, wobei der Bedienvorrichtung eine erste Haptik-Rückkopplungseinheit zugeordnet ist, die dazu ausgebildet ist, eine auf Grundlage einer Abweichung zwischen dem durch manuelle Betätigung des Manipulationsglieds (22) aktuell eingestellten Wert des Beatmungsparameters (110, 112, 114, 116; 112, 110, 112, 114, 116, 120, 122, 130) und dem von der Steuereinheit (100) bestimmten Sollwert des Beatmungsparameters (110, 112, 114, 116; 112, 110, 112, 114, 116, 120, 122, 130) bestimmte erste haptische Rückkopplung zu erzeugen.

2. Beatmungsvorrichtung (10) nach Anspruch 1, wobei die erste Haptik-Rückkopplungseinheit dazu ausgebildet ist, das Manipulationsglied (22) im Sinne einer ersten haptischen Rückkopplung derart anzusteuern, dass eine dem von der Steuereinheit (100) bestimmten Sollwert des wenigstens einen Beatmungsparameters (110, 112, 114, 116; 112, 110, 112, 114, 116, 120, 122, 130) entsprechende Stellung des Manipulationsglieds (22) für die Bedienperson wahrnehmbar hervorgehoben wird.

3. Beatmungsvorrichtung (10) nach Anspruch 2, wobei die Steuereinheit (100) dazu ausgebildet ist, den Sollwert für den wenigstens einen Beatmungsparameter (110, 112, 114, 116; 112, 110, 112, 114, 116, 120, 122, 130) fortlaufend, insbesondere synchronisiert mit jeweiligen Atemzyklen, automatisiert zu bestimmen; und/oder
wobei die erste Haptik-Rückkopplungseinheit dazu ausgebildet ist, das Manipulationsglied (22) derart anzusteuern, dass die dem von der Steuereinheit (100) bestimmten Sollwert des wenigstens einen Beatmungsparameters (110, 112, 114, 116; 112, 110, 112, 114, 116, 120, 122, 130) entsprechende Stellung des Manipulationsglieds (22) für die Bedienperson fühlbar hervorgehoben wird.

4. Beatmungsvorrichtung (10) nach einem der Ansprüche 1 bis 3, wobei die erste Haptik-Rückkopplungseinheit das Manipulationsglied (22) in einer solchen Weise beaufschlagt, dass das Manipulationsglied (22) ohne manuelle Betätigung eine Stellung einnimmt - oder jedenfalls einzunehmen sucht -, die dem durch die Steuereinheit (100) bestimmten Sollwert des Beatmungsparameters (110, 112, 114, 116; 112, 110, 112, 114, 116, 120, 122, 130) entspricht; und/oder
wobei die erste Haptik-Rückkopplungseinheit das Manipulationsglied (22) in einer solchen Weise beaufschlagt, dass das Manipulationsglied (2) in eine Stellung zurückkehrt - oder jedenfalls in eine solche Stellung zurückzukehren sucht -, die dem durch die Steuereinheit (100) bestimmten Sollwert des Beatmungsparameters (110, 112, 114, 116; 112, 110, 112, 114, 116, 120, 122, 130) entspricht, wenn nach erfolgter manueller Betätigung das Manipulationsglied (22) wieder freigegeben wird.

5. Beatmungsvorrichtung (10) nach Anspruch 4, wobei die erste haptische Rückkopplung das Manipulationsglied (22) mit einer Rückstellkraft oder einem Rückstellmoment mit der Charakteristik einer um eine Ruhestellung ausgelenkten Feder, insbesondere einer um einen Ruhepunkt ausgelenkten harmonischen Feder, beaufschlagt.

6. Beatmungsvorrichtung (10) nach einem der Ansprüche 2 bis 5, wobei die erste Haptik-Rückkopplungseinheit dazu ausgebildet ist, im Zuge eines manuellen Manipulationsvorgangs des Manipulationsglieds (22) bei Überfahren einer Stellung des Manipulationsglieds (22), die dem von der Steuereinheit bestimmten Sollwert des Beatmungsparameters entspricht, eine abrupte Änderung der ersten haptischen Rückkopplung zu erzeugen.

7. Beatmungsvorrichtung (10) nach einem der Ansprüche 1 bis 6, wobei die erste Haptik-Rückkopplung davon abhängig ist, in welchem von mehreren Wertebereichen für den Beatmungsparameter (110, 112, 114, 116; 112, 110, 112, 114, 116, 120, 122, 130) sich die Abweichung des durch manuelle Betätigung des Manipulationsglieds (22) aktuell eingestellten Wertes des Beatmungsparameters (110, 112, 114, 116; 112, 110, 112, 114, 116, 120, 122, 130) von dem von der Steuereinheit (100) bestimmten Sollwert des Beatmungsparameters (110, 112, 114, 116; 112, 110, 112, 114, 116, 120, 122, 130) befindet;
wobei insbesondere die mehreren Wertebereiche für den Beatmungsparameter (110, 112, 114, 116; 112, 110, 112, 114, 116, 120, 122, 130) durch jeweilige Wertegrenzen voneinander getrennt sind und die Steuereinheit (100) dazu ausgebildet ist, die Wertegrenzen zwischen den jeweiligen Wertebereichen für den Beatmungsparameter (110, 112, 114, 116; 112, 110, 112, 114, 116, 120, 122, 130) fortlaufend, insbesondere synchronisiert mit jeweiligen Atemzyklen, zu bestimmen.

8. Beatmungsvorrichtung (10) nach einem der Ansprüche 1 bis 7, wobei der ersten Haptik-Rückkopplungseinheit wenigstens ein Sensor zugeordnet ist, der zur Erfassung der Stellung des Manipulationsglieds (22), insbesondere in Bezug auf eine Referenzstellung, ausgebildet ist; und/oder
wobei dem Manipulationsglied (22) eine zweite Haptik-Rückkopplungseinheit zugeordnet ist, welche das Manipulationsglied (22) abhängig von einem Ist-Zustand des wenigstens einen Beatmungsparameters (110, 112, 114, 116; 112, 110, 112, 114, 116, 120, 122, 130) im Sinne einer zweiten haptischen Rückkopplung beaufschlagt.

9. Beatmungsvorrichtung (10) nach Anspruch 8, wobei die zweite Haptik-Rückkopplungseinheit das Manipulationsglied (22) mit einer den Ist-Zustand des wenigstens einen Beatmungsparameters (110, 112, 114, 116; 112, 110, 112, 114, 116, 120, 122, 130) anzeigenden zweiten haptischen Rückkopplung beaufschlagt; und/oder
wobei der zweiten Haptik-Rückkopplungseinheit wenigstens ein Sensor zugeordnet ist, der zur Erfassung des wenigstens einen Beatmungsparameters (110, 112, 114, 116; 112, 110, 112, 114, 116, 120, 122, 130) ausgebildet ist.

10. Beatmungsvorrichtung (10) nach einem der Ansprüche 1 bis 9, ferner umfassend wenigstens einen Sensor zur Erfassung wenigstens einer der folgenden Kenngrößen: aktueller Atemwegsdruck am Atemwegseingang, aktueller Gasfluss am Atemwegseingang, kumulierter Gasfluss im Atemweg während der Inspirationsphase eines Atemzyklus, kumulierter Gasfluss im Atemweg während der Expirationsphase eines Atemzyklus, Kohlendioxidkonzentration im vom Patienten am Ende eines Atemzyklus ausgeatmeten Atemgas, arterielle Sauerstoffsättigung im Blutkreislauf des Patienten; und/oder
wobei die Steuereinheit (100) dazu ausgebildet ist, auf Grundlage der erfassten Werte von Kenngrößen einen Beatmungsmodus aus einer Mehrzahl von vorgegebenen Beatmungsmodi auszuwählen und auf Grundlage des ausgewählten Beatmungsmodus den Sollwert für den wenigstens einen Beatmungsparameter (110, 112, 114, 116; 112, 110, 112, 114, 116, 120, 122, 130) automatisiert zu bestimmen; und/oder
wobei der wenigstens eine Beatmungsparameter (110, 112, 114, 116; 112, 110, 112, 114, 116, 120, 122, 130) wenigstens einen der folgenden Parameter umfasst: Beatmungsfrequenz (110), Tidalvolumen (112), Minutenvolumen (122), Inspirationszeit (114), positiver endexpiratorischer Druck (PEEP, 120), maximaler Atemwegsdruck (116), Sauerstoffkonzentration im dem Patienten zugeführten Atemgas (130); und/oder
wobei das Manipulationsglied (22) als Drehglied ausgebildet ist, ein als Drehglied ausgebildetes Einstellelement umfasst; oder
als Dreh-/Drück-Steller ausgebildet ist.

11. Beatmungsvorrichtung (10) nach einem der Ansprüche 1 bis 10, wobei das Manipulationsglied (22) zur Einstellung von mehreren Beatmungsparametern (110, 112, 114, 116; 112, 110, 112, 114, 116, 120, 122, 130) ausgebildet ist.

12. Verfahren zum Bereitstellen einer unterstützenden Interaktion beim Einstellen wenigstens eines Parameters (110, 112, 114, 116; 112, 110, 112, 114, 116, 120, 122, 130) bei einer Beatmungsvorrichtung (10), bei welchem
- ein Manipulationsglied (22) einer Bedienvorrichtung zum Einstellen des wenigstens einen Parameters (110, 112, 114, 116; 112, 110, 112, 114, 116, 120, 122, 130) durch eine Bedienperson manuell betätigt wird, und
- auf Grundlage von erfassten Werten von Kenngrößen ein Sollwert für den wenigstens einen Parameter (110, 112, 114, 116; 112, 110, 112, 114, 116, 120, 122, 130) durch eine Steuereinheit (100) automatisiert bestimmt wird,
wobei das Manipulationsglied (22) während der manuellen Betätigung des Manipulationsglieds (22) mit einer auf Grundlage einer Abweichung zwischen dem durch manuelle Betätigung des Manipulationsglieds (22) aktuell eingestellten Wert des Parameters (110, 112, 114, 116; 112, 110, 112, 114, 116, 120, 122, 130) und dem von der Steuereinheit (100) bestimmten Sollwert des Parameters (110, 112, 114, 116; 112, 110, 112, 114, 116, 120, 122, 130) bestimmten ersten haptischen Rückkopplung beaufschlagt wird.

13. Verfahren nach Anspruch 12, wobei das Manipulationsglied (2) während der manuellen Betätigung im Sinne der ersten haptischen Rückkopplung derart angesteuert wird, dass eine dem von der Steuereinheit (100) bestimmten Sollwert des wenigstens einen Parameters (110, 112, 114, 116; 112, 110, 112, 114, 116, 120, 122, 130) entsprechende Stellung des Manipulationsglieds (22) für die Bedienperson wahrnehmbar, insbesondere fühlbar, hervorgehoben wird.

14. Verfahren nach Anspruch 12 oder 13, wobei das Manipulationsglied (22) in einer solchen Weise beaufschlagt wird, dass das Manipulationsglied (22) ohne manuelle Betätigung eine Stellung einnimmt - oder jedenfalls einzunehmen sucht -, die dem durch die Steuereinheit (100) bestimmten Sollwert des Parameters (110, 112, 114, 116; 112, 110, 112, 114, 116, 120, 122, 130) entspricht; und/oder
wobei das Manipulationsglied (22) in einer solchen Weise beaufschlagt wird, dass das Manipulationsglied (22) in eine Stellung zurückkehrt - oder jedenfalls in eine solche Stellung zurückzukehren sucht -, die dem durch die Steuereinheit (100) bestimmten Sollwert des Parameters (110, 112, 114, 116; 112, 110, 112, 114, 116, 120, 122, 130) entspricht, wenn nach erfolgter manueller Betätigung das Manipulationsglied (22) wieder freigegeben wird; und/oder
wobei die das Manipulationsglied (22) mit einer Rückstellkraft oder einem Rückstellmoment mit der Charakteristik einer um eine Ruhestellung ausgelenkten Feder, insbesondere einer um einen Ruhepunkt ausgelenkten harmonischen Feder, beaufschlagt wird.

15. Computerprogrammprodukt, welches Programmanweisungen enthält, bei deren Ausführung auf der Steuereinheit der Beatmungsvorrichtung nach Anspruch 1 ein Verfahren nach einem der Ansprüche 12 bis 14 ausgeführt wird.

## Claims

1. A ventilation device (10) for mechanical ventilation of patients,
comprising
- an operating device for setting at least one ventilation parameter (110, 112, 114, 116; 112, 110, 112, 114, 116, 120, 122, 130), having a manipulation element (22) which can be manually operated by an operator for manually setting the at least one ventilation parameter (110, 112, 114, 116; 112, 110, 112, 114, 116, 120, 122, 130), and
- a control unit (100) which is designed to automatically determine a target value for the at least one ventilation parameter (110, 112, 114, 116; 112, 110, 112, 114, 116, 120, 122, 130) on the basis of detected values of characteristic variables,
wherein the operating device has a first haptic feedback unit associated therewith which is designed to generate a first haptic feedback determined on the basis of a deviation between the value of the ventilation parameter (110, 112, 114, 116; 112, 110, 112, 114, 116, 120, 122, 130) currently set by manual operation of the manipulation element (22) and the target value of the ventilation parameter (110, 112, 114, 116; 112, 110, 112, 114, 116, 120, 122, 130) determined by the control unit (100).

2. The ventilation device (10) of claim 1,
wherein the first haptic feedback unit is designed to control the manipulation element (22) in terms of a first haptic feedback such that a position of the manipulation element (22) corresponding to the target value of the at least one ventilation parameter (110, 112, 114, 116; 112, 110, 112, 114, 116, 120, 122, 130) determined by the control unit (100) is emphasized so as to be perceptible for the operator.

3. The ventilation device (10) of claim 2,
wherein the control unit (100) is designed to automatically determine the target value for the at least one ventilation parameter (110, 112, 114, 116; 112, 110, 112, 114, 116, 120, 122, 130) in continuous manner, in particular synchronized with respective breathing cycles; and/or
wherein the first haptic feedback unit is designed to control the manipulation element (22) such that the position of the manipulation element (22) corresponding to the target value of the at least one ventilation parameter (110, 112, 114, 116; 112, 110, 112, 114, 116, 120, 122, 130) determined by the control unit (100) is emphasized so as to be tangible for the operator.

4. The ventilation device (10) of any of claims 1 to 3,
wherein the first haptic feedback unit acts on the manipulation element (22) in such a way that the manipulation element (22), without manual operation thereof, assumes a position - or in any case tries to assume a position - corresponding to the target value of the ventilation parameter (110, 112, 114, 116; 112, 110, 112, 114, 116, 120, 122, 130) determined by the control unit (100); and/or
wherein the first haptic feedback unit acts on the manipulation element (22) in such a way that the manipulation element (2) returns to a position - or in any case tries to return to a position - corresponding to the target value of the ventilation parameter (110, 112, 114, 116; 112, 110, 112, 114, 116, 120, 122, 130) determined by the control unit (100) when the manipulation element (22) is released again upon effected manual operation thereof.

5. The ventilation device (10) of claim 4,
wherein the first haptic feedback applies to the manipulation element (22) a restoring force or a restoring moment having the characteristic of a spring that is deflected around a rest position, in particular a harmonic spring deflected around a point of rest.

6. The ventilation device (10) of any of claims 2 to 5,
wherein the first haptic feedback unit is designed to generate an abrupt change in the first haptic feedback in the course of a manual manipulation process of the manipulation element (22) when a position of the manipulation element (22) is passed which corresponds to the target value of the ventilation parameter determined by the control unit.

7. The ventilation device (10) of any of claims 1 to 6,
wherein the first haptic feedback is dependent on which one of several value ranges for the ventilation parameter (110, 112, 114, 116; 112, 110, 112, 114, 116, 120, 122, 130) involves the deviation of the value of the ventilation parameter (110, 112, 114, 116; 112, 110, 112, 114, 116, 120, 122, 130) currently set by manual operation of the manipulation element (22) from the target value of the ventilation parameter (110, 112, 114, 116; 112, 110, 112, 114, 116, 120, 122, 130) determined by the control unit (100);
wherein in particular the several value ranges for the ventilation parameter (110, 112, 114, 116; 112, 110, 112, 114, 116, 120, 122, 130) are separated from one another by respective value limits, and the control unit (100) is designed to determine the value limits between the respective value ranges for the ventilation parameter (110, 112, 114, 116; 112, 110, 112, 114, 116, 120, 122, 130) continuously, in particular synchronized with respective breathing cycles.

8. The ventilation device (10) of any of claims 1 to 7,
wherein the first haptic feedback unit has at least one sensor associated therewith which is designed to detect the position of the manipulation element (22), in particular with respect to a reference position; and/or
wherein the manipulation element (22) has a second haptic feedback unit associated therewith which acts on the manipulation element (22) as a function of an actual state of the at least one ventilation parameter (110, 112, 114, 116; 112, 110, 112, 114, 116, 120, 122, 130) in terms of a second haptic feedback.

9. The ventilation device (10) of claim 8,
wherein the second haptic feedback unit applies to the manipulation element (22) a second haptic feedback which indicates the actual state of the at least one ventilation parameter (110, 112, 114, 116; 112, 110, 112, 114, 116, 120, 122, 130); and/or
wherein the second haptic feedback unit has at least one sensor associated therewith which is designed to detect the at least one ventilation parameter (110, 112, 114, 116; 112, 110, 112, 114, 116, 120, 122, 130).

10. The ventilation device (10) of any of claims 1 to 9,
further comprising at least one sensor for detecting at least one of the following characteristic variables: current airway pressure at the airway entrance, current gas flow at the airway entrance, accumulated gas flow in the airway during the inspiration phase of a breathing cycle, accumulated gas flow in the airway during the expiration phase of a breathing cycle, carbon dioxide concentration in the breathing gas exhaled by the patient at the end of a breathing cycle, arterial oxygen saturation in the patient's bloodstream; and/or
wherein the control unit (100) is designed to select a ventilation mode from a plurality of predetermined ventilation modes on the basis of the detected values of characteristic variables and to automatically determine the target value for the at least one ventilation parameter (110, 112, 114, 116; 112, 110, 112, 114, 116, 120, 122, 130) on the basis of the ventilation mode selected; and/or
wherein the at least one ventilation parameter (110, 112, 114, 116; 112, 110, 112, 114, 116, 120, 122, 130) comprises at least one of the following parameters: ventilation frequency (110), tidal volume (112), minute volume (122), inspiration time (114), positive end-expiratory pressure (PEEP, 120), maximum airway pressure (116), oxygen concentration in the breathing gas supplied to the patient (130); and/or
wherein the manipulation element (22) is designed as a rotary member, comprises an adjusting element formed as a rotary member; or
is designed as a rotary/push-type actuator.

11. The ventilation device (10) of any of claims 1 to 10,
wherein the manipulation element (22) is designed for setting a plurality of ventilation parameters (110, 112, 114, 116; 112, 110, 112, 114, 116, 120, 122, 130).

12. A method for providing a supportive interaction in setting at least one parameter (110, 112, 114, 116; 112, 110, 112, 114, 116, 120, 122, 130) of a ventilation device (10), wherein
- a manipulation element (22) of an operating device for setting the at least one parameter (110, 112, 114, 116; 112, 110, 112, 114, 116, 120, 122, 130) is operated manually by an operator, and
- on the basis of detected values of characteristic variables, a target value for the at least one parameter (110, 112, 114, 116; 112, 110, 112, 114, 116, 120, 122, 130) is determined automatically by a control unit (100),
wherein the manipulation element (22), during manual operation of the manipulation element (22), is acted upon with a first haptic feedback which is determined on the basis of a deviation between the value of the parameter (110, 112, 114, 116; 112, 110, 112, 114, 116, 120, 122) currently set by manual operation of the manipulation element (22) and the target value of the parameter (110, 112, 114, 116; 112, 110, 112, 114, 116, 120, 122, 130) determined by the control unit (100).

13. The method of claim 12,
wherein the manipulation element (2), during manual operation thereof, is controlled in terms of a first haptic feedback such that a position of the manipulation element (22) corresponding to the target value of the at least one parameter (110, 112, 114, 116; 112, 110, 112, 114, 116, 120, 122, 130) determined by the control unit (100) is emphasized so as to be perceptible, in particular tangible, for the operator.

14. The method of claim 12 or 13,
wherein the manipulation element (22) is acted upon in such a way that the manipulation element (22), without manual operation thereof, assumes a position - or in any case tries to assume a position - corresponding to the target value of the parameter (110, 112, 114, 116; 112, 110, 112, 114, 116, 120, 122, 130) determined by the control unit (100); and/or
wherein the manipulation element (22) is acted upon in such a way that the manipulation element (22) returns to a position - or in any case tries to return to a position - corresponding to the target value of the parameter (110, 112, 114, 116; 112, 110, 112, 114, 116, 120, 122, 130) determined by the control unit (100) when the manipulation element (22) is released again upon effected manual operation thereof; and/or
wherein the manipulation element (22) is acted upon with a restoring force or a restoring moment having the characteristic of a spring that is deflected around a rest position, in particular a harmonic spring deflected around a point of rest.

15. A computer program product which contains program instructions, the execution of which on the control unit of the ventilation device according to claim 1 implements a method according to any of claims 12 to 14.

## Revendications

1. Dispositif du type à ventilation mécanique assistée (10) destiné à la ventilation mécanique de patients, qui comprend :
- un dispositif d'exploitation destiné au réglage d'au moins un paramètre de la ventilation mécanique (110, 112, 114, 116 ; 112, 110, 112, 114, 116, 120, 122, 130), qui comprend un organe de manipulation (22) qui peut être actionné manuellement par un opérateur à des fins de réglage manuel dudit au moins un paramètre de la ventilation mécanique (110, 112, 114, 116 ; 112, 110, 112, 114, 116, 120, 122, 130) ; et
- une unité de commande (100) qui est réalisée dans le but de déterminer de manière automatique, sur la base de valeurs enregistrées de caractéristiques, une valeur de consigne pour ledit au moins un paramètre de la ventilation mécanique (110, 112, 114, 116 ; 112, 110, 112, 114, 116, 120, 122, 130) ; dans lequel une première unité de rétroaction tactile est attribuée au dispositif d'exploitation, unité qui est réalisée dans le but de générer, sur base d'une déviation entre la valeur réglée en vigueur, par l'intermédiaire d'une activation manuelle de l'organe de manipulation (22), du paramètre de la ventilation mécanique (110, 112, 114, 116 ; 112, 110, 112, 114, 116, 120, 122, 130), et la valeur de consigne du paramètre de la ventilation mécanique (110, 112, 114, 116; 112, 110, 112, 114, 116, 120, 122, 130) qui a été déterminée par l'unité de commande (100), une première rétroaction tactile déterminée.

2. Dispositif du type à ventilation mécanique assistée (10) conformément à la revendication 1, dans lequel la première unité de rétroaction tactile est réalisée dans le but de déclencher l'organe de manipulation (22) dans le sens d'une première rétroaction tactile, d'une manière telle qu'une position de l'organe de manipulation (22) qui correspond à la valeur de consigne, qui a été déterminée par l'unité de commande (100), dudit au moins un paramètre de la ventilation mécanique (110, 112, 114, 116; 112, 110, 112, 114, 116, 120, 122, 130), est mise en évidence de manière perceptible pour l'opérateur.

3. Dispositif du type à ventilation mécanique assistée (10) conformément à la revendication 2, dans lequel l'unité de commande (100) est réalisée dans le but de déterminer de manière automatique la valeur de consigne pour ledit au moins un paramètre de la ventilation mécanique (110, 112, 114, 116 ; 112, 110, 112, 114, 116, 120, 122, 130) en continu, en particulier de manière synchronisée avec chaque cycle respiratoire ; et/ou dans lequel la première unité de rétroaction tactile est réalisée dans le but de déclencher l'organe de manipulation (22) d'une manière telle que la position de l'organe de manipulation (22) qui correspond à la valeur de consigne dudit au moins un paramètre de la ventilation mécanique (110, 112, 114, 116 ; 112, 110, 112, 114, 116, 120, 122, 130), qui a été déterminée par l'unité de commande (100), est mise en évidence de manière tangible pour l'opérateur.

4. Dispositif du type à ventilation mécanique assistée (10) conformément à l'une quelconque des revendications 1 à 3, dans lequel la première unité de rétroaction tactile sollicite l'organe de manipulation (22) d'une manière telle que l'organe de manipulation (22) prend une position - ou en tout cas cherche à prendre une position -, en l'absence d'un déclenchement manuel, qui correspond à la valeur de consigne du paramètre de la ventilation mécanique (110, 112, 114, 116 ; 112, 110, 112, 114, 116, 120, 122, 130), qui a été déterminée par l'unité de commande (100) ; et/ou dans lequel la première unité de rétroaction tactile sollicite l'organe de manipulation (22) d'une manière telle que l'organe de manipulation (22) reprend une position - ou en tout cas cherche à reprendre une telle position - qui correspond à la valeur de consigne du paramètre de la ventilation mécanique (110, 112, 114, 116 ; 112, 110, 112, 114, 116, 120, 122, 130), qui a été déterminée par l'unité de commande (100) lorsque, après une activation manuelle réussie, l'organe de manipulation (22) est à nouveau libéré.

5. Dispositif du type à ventilation mécanique assistée (10) conformément à la revendication 4, dans lequel la première unité de rétroaction tactile sollicite l'organe de manipulation (22) avec une force de rétroaction ou avec un moment de rétroaction avec la caractéristique d'un ressort allongé par rapport à sa position de repos, en particulier d'un ressort harmonique allongé par rapport à sa position de repos.

6. Dispositif du type à ventilation mécanique assistée (10) conformément à l'une quelconque des revendications 2 à 5, dans lequel la première unité de rétroaction tactile est réalisée dans le but de générer, à la suite d'un processus d'activation manuelle de l'organe de manipulation (22) dans le cas d'un dépassement d'une position de l'organe de manipulation (22), qui correspond à la valeur de consigne du paramètre de la ventilation mécanique qui a été déterminée par l'unité de commande, une modification brusque de la première rétroaction tactile.

7. Dispositif du type à ventilation mécanique assistée (10) conformément à l'une quelconque des revendications 1 à 6, dans lequel la première unité de rétroaction tactile dépend du fait de savoir dans quelle plage parmi plusieurs plages de valeurs pour le paramètre de la ventilation mécanique (110, 112, 114, 116 ; 112, 110, 112, 114, 116, 120, 122, 130) se trouve la déviation de la valeur du paramètre de la ventilation mécanique (110, 112, 114, 116 ; 112, 110, 112, 114, 116, 120, 122, 130) en vigueur qui a été réglée par l'intermédiaire de l'activation manuelle de l'organe de manipulation (22) par rapport à la valeur de consigne du paramètre de la ventilation mécanique (110, 112, 114, 116; 112, 110, 112, 114, 116, 120, 122, 130) qui a été déterminée par l'unité de commande (100) ; dans lequel, en particulier, lesdites plusieurs plages de valeurs pour le paramètre de la ventilation mécanique (110, 112, 114, 116 ; 112, 110, 112, 114, 116, 120, 122, 130) sont séparées les unes des autres par des valeurs limites respectives, et l'unité de commande (100) est réalisée dans le but de déterminer en continu, en particulier de manière synchronisée avec des cycles de respiration respectifs, les valeurs limites entre les plages de valeurs respectives pour le paramètre de la ventilation mécanique (110, 112, 114, 116 ; 112, 110, 112, 114, 116, 120, 122, 130).

8. Dispositif du type à ventilation mécanique assistée (10) conformément à l'une quelconque des revendications 1 à 7, dans lequel au moins un capteur est attribué à la première unité de rétroaction tactile, qui est réalisé dans le but d'enregistrer la position de l'organe de manipulation (22), en particulier par rapport à une position de référence ; et/ou dans lequel une deuxième unité de rétroaction tactile est attribuée à l'organe de manipulation (22), qui sollicite l'organe de manipulation (22) en fonction d'un état réel dudit au moins un paramètre de la ventilation mécanique (110, 112, 114, 116 ; 112, 110, 112, 114, 116, 120, 122, 130), dans le sens d'une deuxième rétroaction tactile.

9. Dispositif du type à ventilation mécanique assistée (10) conformément à la revendication 8, dans lequel la deuxième unité de rétroaction tactile sollicite l'organe de manipulation (22) avec une deuxième rétroaction tactile qui affiche l'état réel dudit au moins un paramètre de la ventilation mécanique (110, 112, 114, 116 ; 112, 110, 112, 114, 116, 120, 122, 130) ; et/ou dans lequel au moins un capteur est attribué à la deuxième unité de rétroaction tactile, qui est réalisé à des fins d'enregistrement dudit au moins un paramètre de la ventilation mécanique (110, 112, 114, 116; 112, 110, 112, 114, 116, 120, 122, 130).

10. Dispositif du type à ventilation mécanique assistée (10) conformément à l'une quelconque des revendications 1 à 9, qui comprend en outre au moins un capteur qui est destiné à l'enregistrement d'au moins une des caractéristiques suivantes, à savoir : la pression en vigueur qui règne dans les voies respiratoires à l'entrée du système respiratoire ; le flux de gaz en vigueur à l'entrée du système respiratoire ; le flux de gaz cumulé dans les voies respiratoires au cours de la phase d'inspiration d'un cycle de respiration ; le flux de gaz cumulé dans les voies respiratoires au cours de la phase d'expiration d'un cycle de respiration ; la concentration de dioxyde de carbone dans le gaz de respiration expiré par le patient à la fin d'un cycle de respiration ; la saturation artérielle en oxygène dans le système sanguin du patient ; et/ou dans lequel l'unité de commande (100) est réalisée dans le but de sélectionner d'une manière automatique, sur la base des valeurs enregistrées de caractéristiques, un mode de respiration à partir d'une multitude de modes de respiration qui ont été prédéfinis et dans le but de déterminer de manière automatique, sur la base du mode de respiration qui a été sélectionné, la valeur de consigne pour ledit au moins un paramètre de la ventilation mécanique (110, 112, 114, 116 ; 112, 110, 112, 114, 116, 120, 122, 130) ; et/ou dans lequel ledit au moins un paramètre de la ventilation mécanique (110, 112, 114, 116 ; 112, 110, 112, 114, 116, 120, 122, 130) comprend au moins un des paramètres indiqués ci-après, à savoir : le rythme respiratoire (110), le volume courant (112), le débit-volume (122), la durée de l'inspiration (114), la pression positive en fin d'expiration (PEEP, 120), la pression maximale qui règne dans les voies respiratoires (116), la concentration d'oxygène dans le gaz respiratoire qui alimente le patient (130) ; et/ou dans lequel l'organe de manipulation (22) est réalisé sous la forme d'un organe rotatif, comprend un élément de réglage qui est réalisé sous la forme d'un organe rotatif ou est réalisé sous la forme d'un actionneur par rotation/pression.

11. Dispositif du type à ventilation mécanique assistée (10) conformément à l'une quelconque des revendications 1 à 10, dans lequel l'organe de manipulation (22) est réalisé à des fins de réglage de plusieurs paramètres de la ventilation mécanique (110, 112, 114, 116; 112, 110, 112, 114, 116, 120, 122, 130).

12. Procédé destiné à la mise à disposition d'une interaction procurant un soutien lors du réglage d'au moins un paramètre (110, 112, 114, 116; 112, 110, 112, 114, 116, 120, 122, 130) dans le cadre d'un appareil du type à ventilation mécanique assistée (10) ; dans lequel
- on actionne à la main un organe de manipulation (22) d'un dispositif d'exploitation à des fins de réglage dudit au moins un paramètre (110, 112, 114, 116; 112, 110, 112, 114, 116, 120, 122, 130) par l'intermédiaire d'un opérateur ; et
- sur la base de valeurs enregistrées de caractéristiques, on détermine de manière automatique une valeur de consigne pour ledit au moins un paramètre (110, 112, 114, 116 ; 112, 110, 112, 114, 116, 120, 122, 130) par l'intermédiaire d'une unité de commande (100) ;
dans lequel l'organe de manipulation (22), au cours de l'activation manuelle de l'organe de manipulation (22) est sollicité avec une première rétroaction tactile qui a été déterminée, sur la base d'une déviation entre la valeur du paramètre (110, 112, 114, 116 ; 112, 110, 112, 114, 116, 120, 122, 130) réglée en vigueur par l'intermédiaire d'une activation manuelle de l'organe de manipulation (22) et la valeur de consigne du paramètre (110, 112, 114, 116 ; 112, 110, 112, 114, 116, 120, 122, 130) qui a été déterminée par l'unité de commande (100).

13. Procédé conformément à la revendication 12, dans lequel l'organe de manipulation (2) est excité, au cours de l'activation manuelle dans le sens de la première rétroaction tactile, d'une manière telle qu'une position de l'organe de manipulation (22) qui correspond à la valeur de consigne dudit au moins un paramètre (110, 112, 114, 116; 112, 110, 112, 114, 116, 120, 122, 130), qui a été déterminée par l'unité de commande (100), est mise en évidence de manière perceptible, en particulier de manière tangible pour l'opérateur.

14. Procédé conformément à la revendication 12 ou 13, dans lequel l'organe de manipulation (22) est sollicité d'une manière telle que l'organe de manipulation (22) prend une position - ou en tout cas cherche à prendre une position - en l'absence d'un déclenchement manuel, qui correspond à la valeur de consigne du paramètre (110, 112, 114, 116; 112, 110, 112, 114, 116, 120, 122, 130), qui a été déterminée par l'unité de commande (100) ; et/ou dans lequel l'organe de manipulation (22) est sollicité d'une manière telle que l'organe de manipulation (22) reprend une position - ou en tout cas cherche à reprendre une telle position - qui correspond à la valeur de consigne du paramètre (110, 112, 114, 116 ; 112, 110, 112, 114, 116, 120, 122, 130), qui a été déterminée par l'unité de commande (100) lorsque, à la suite d'une activation manuelle réussie, l'organe de manipulation (22) est à nouveau libéré; et/ou dans lequel l'organe de manipulation (22) est sollicité avec une force de rétroaction ou avec un moment de rétroaction avec la caractéristique d'un ressort allongé par rapport à une position de repos, en particulier d'un ressort harmonique allongé par rapport à une position de repos.

15. Produit de programme informatique qui contient des instructions de programme en fonction desquelles, lorsque celles-ci sont exécutées sur l'unité de commande de l'appareil du type à ventilation mécanique assistée conformément à la revendication 1, un procédé conformément à l'une quelconque des revendications 12 à 14 est mis en oeuvre.
